(19) Europäisches Patentamt
European Patent Office
Office européen des brevets

(11) EP 4 481 050 A1

(12) EUROPEAN PATENT APPLICATION
published in accordance with Art. 153(4) EPC

(43) Date of publication:
**25.12.2024 Bulletin 2024/52**

(21) Application number: **23756341.6**

(22) Date of filing: **14.02.2023**

(51) International Patent Classification (IPC):
***C12P 7/42*** *(2006.01)* ***C12N 1/21*** *(2006.01)*
***C12N 9/02*** *(2006.01)* ***C12N 9/04*** *(2006.01)*
***C12N 15/53*** *(2006.01)* ***C12N 15/60*** *(2006.01)*

(52) Cooperative Patent Classification (CPC):
**C12N 9/0004; C12N 9/88; C12N 15/74; C12P 7/42**

(86) International application number:
**PCT/JP2023/004887**

(87) International publication number:
**WO 2023/157816 (24.08.2023 Gazette 2023/34)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **15.02.2022 JP 2022020966**

(71) Applicant: **Toray Industries, Inc.**
**Tokyo 103-8666 (JP)**

(72) Inventors:
- **MORITA, Keisuke**
  **Kamakura-shi, Kanagawa 248-8555 (JP)**
- **ISOBE, Kyohei**
  **Kamakura-shi, Kanagawa 248-8555 (JP)**
- **KAWAMURA, Kenji**
  **Kamakura-shi, Kanagawa 248-8555 (JP)**
- **YAMADA, Katsushige**
  **Kamakura-shi, Kanagawa 248-8555 (JP)**

(74) Representative: **Kador & Partner Part mbB**
**Corneliusstraße 15**
**80469 München (DE)**

Remarks:
The complete document including Reference Table(s) and the Sequence Listing(s) can be downloaded from the EPO website

# (54) GENETICALLY MODIFIED MICROORGANISM FOR PRODUCING 3-HYDROXYADIPIC ACID AND/OR ALPHA-HYDROMUCONIC ACID, AND METHOD FOR PRODUCING CHEMICAL PRODUCT

(57) Disclosed is a novel genetically modified microorganism showing improved yields of 3-hydroxyadipic acid and/or α-hydromuconic acid. The genetically modified microorganism is a microorganism having an ability to produce 3-hydroxyadipic acid and/or α-hydromuconic acid, in which the reaction to generate malic acid from oxaloacetic acid is enhanced, and the reaction to generate acetyl-CoA from pyruvic acid is enhanced. In addition, the reaction to generate carbon dioxide from formic acid is enhanced.

EP 4 481 050 A1

## Description

TECHNICAL FIELD

The present invention relates to a genetically modified microorganism that produces 3-hydroxyadipic acid and/or α-hydromuconic acid in high yields, a method of producing 3-hydroxyadipic acid and/or α-hydromuconic acid using the genetically modified microorganism, and so on.

BACKGROUND ART

3-Hydroxyadipic acid (IUPAC name: 3-hydroxyhexanedioic acid) and α-hydromuconic acid (IUPAC name: (E)-hex-2-enedioic acid) are dicarboxylic acids containing six carbon atoms. These dicarboxylic acids can be used as raw materials for the production of polyesters by polymerization with polyols or as raw materials for the production of polyamides by polymerization with polyamines. Additionally, compounds obtained by adding ammonia to the end of these dicarboxylic acids and converting the resultants to lactams can also be used as raw materials for polyamides.

Patent Document 1, as a document concerning the production of 3-hydroxyadipic acid and/or α-hydromuconic acid using genetically modified microorganisms having a modified metabolic pathway, describes a method of producing 3-hydroxyadipic acid, α-hydromuconic acid, and/or adipic acid by using polypeptide showing excellent catalytic activity in the reduction reaction from 3-oxoadipyl-CoA to 3-hydroxyadipyl-CoA. It is described in the document that the biosynthesis pathway for these substances proceeds through an enzymatic reaction that reduces 3-oxoadipyl-CoA to 3-hydroxyadipyl-CoA. Patent Document 2 also describes a method of producing 3-hydroxyadipic acid, α-hydromuconic acid, and/or adipic acid by using polypeptide showing excellent catalytic activity in the reduction reaction from 3-oxoadipyl-CoA to 3-hydroxyadipyl-CoA, as well as by using a genetically modified microorganism having defective pyruvate kinase function.

Patent Document 3 describes a method of producing 3-hydroxyadipic acid, α-hydromuconic acid, and/or adipic acid by using a genetically modified microorganism that is a microorganism having an ability to produce 3-hydroxyadipic acid, α-hydromuconic acid, and/or adipic acid, which lacks the function of pyruvate kinase, has defective functions of phosphotransferase enzymes, and has enhanced activity of phosphoenolpyruvate carboxykinase.

PRIOR ART DOCUMENTS

PATENT DOCUMENTS

Patent Document 1: WO 2019/107516
Patent Document 2: WO 2020/230718
Patent Document 3: WO 2020/230719

SUMMARY OF THE INVENTION

PROBLEMS TO BE SOLVED BY THE INVENTION

As described above, for production of 3-hydroxyadipic acid and/or α-hydromuconic acid using a microorganism having an ability to produce 3-hydroxyadipic acid and/or α-hydromuconic acid, a technology has been established where a metabolic pathway of the microorganism is modified to increase the yield(s) of 3-hydroxyadipic acid and/or α-hydromuconic acid. Modification of a previously unknown metabolism-related gene contributing to the increase in the yield(s) of 3-hydroxyadipic acid and/or α-hydromuconic acid can be expected to further increase the yield(s).

Accordingly, the present invention aims to provide a novel genetically modified microorganism in which the yield(s) of 3-hydroxyadipic acid and/or α-hydromuconic acid is/are increased by identifying a previously unknown metabolism-related gene contributing to the increase in the yield(s) of 3-hydroxyadipic acid and/or α-hydromuconic acid, and modifying a metabolic pathway involving the metabolism-related gene.

MEANS FOR SOLVING THE PROBLEMS

The present inventors have intensively studied in order to achieve the object described above and consequently found that, in a microorganism having an ability to produce 3-hydroxyadipic acid and/or α-hydromuconic acid, enhancement of the reaction to generate malic acid from oxaloacetic acid and enhancement of the reaction to generate acetyl-CoA from pyruvic acid in a metabolic pathway by genetic modification result in increase in the yield(s) of 3-hydroxyadipic acid

and/or α-hydromuconic acid, thereby completing the present invention.

That is, the present invention provides the following (1) to (15):

(1) A genetically modified microorganism having an ability to produce 3-hydroxyadipic acid and/or α-hydromuconic acid, wherein the reaction to generate malic acid from oxaloacetic acid is enhanced, and the reaction to generate acetyl-CoA from pyruvic acid is enhanced.
(2) The genetically modified microorganism of (1), wherein the enhancement of the reaction to generate malic acid from oxaloacetic acid is an enhancement of the reaction catalyzed by malate dehydrogenase.
(3) The genetically modified microorganism of (1) or (2), wherein the enhancement of the reaction to generate acetyl-CoA from pyruvic acid is an enhancement of the reaction catalyzed by pyruvate dehydrogenase complex and/or an enhancement of the reaction catalyzed by pyruvate formate-lyase.
(4) The genetically modified microorganism of (3), wherein the enhancement of the reaction catalyzed by the pyruvate dehydrogenase complex is an enhancement by increased expression of the pyruvate dehydrogenase complex and/or increased activity of the pyruvate dehydrogenase complex.
(5) The genetically modified microorganism of (4), wherein the increased expression of the pyruvate dehydrogenase complex is achieved by reducing the function of transcriptional repressor of the pyruvate dehydrogenase complex.
(6) The genetically modified microorganism of (4), wherein the increased activity of the pyruvate dehydrogenase complex is achieved by reducing the sensitivity of the pyruvate dehydrogenase complex to NADH.
(7) The genetically modified microorganism of (3), wherein the enhancement of the reaction catalyzed by pyruvate formate-lyase is achieved by enhancement by increased expression of pyruvate formate-lyase.
(8) The genetically modified microorganism of any one of (1) to (7), wherein the reaction to generate carbon dioxide from formic acid is enhanced.
(9) The genetically modified microorganism of (8), wherein the enhancement of the reaction to generate carbon dioxide from formic acid is an enhancement of the reaction catalyzed by $NAD^+$-dependent formate dehydrogenase.
(10) The genetically modified microorganism of any one of (1) to (9), wherein the reaction that reduces 3-oxoadipyl-CoA to generate 3-hydroxyadipyl-CoA is further enhanced.
(11) The genetically modified microorganism of any one of (1) to (10), wherein the microorganism is one which does not have glucose metabolism via the phosphoketolase pathway.
(12) A method of producing 3-hydroxyadipic acid and/or α-hydromuconic acid, comprising the step of culturing the genetically modified microorganism of any one of (1) to (11).
(13) A method of producing adipic acid, comprising the step of producing 3-hydroxyadipic acid and/or α-hydromuconic acid by the process of (12), and the step of allowing the 3-hydroxyadipic acid and/or α-hydromuconic acid to react with hydrogen in the presence of a hydrogenation catalyst.
(14) A method of producing polyamide, comprising the step of producing adipic acid by the method of (13), and the step of polycondensation of the adipic acid and a diamine.
(15) The method of producing polyamide of (14), wherein the diamine is one containing 1,4-butanediamine, 1,5-pentanediamine, or hexamethylenediamine.

## EFFECT OF THE INVENTION

A microorganism having an ability to produce 3-hydroxyadipic acid and/or α-hydromuconic acid, the microorganism having genetic modification whereby the reaction to generate malic acid from oxaloacetic acid is enhanced and the reaction to generate acetyl-CoA from pyruvic acid is enhanced, can produce 3-hydroxyadipic acid and/or α-hydromuconic acid in higher yields than the parent microorganism strain without the gene modification. In addition, genetic modification whereby the reaction to generate carbon dioxide from formic acid is enhanced can further increase the productivity for 3-hydroxyadipic acid and/or α-hydromuconic acid.

## DETAILED DESCRIPTION OF THE INVENTION

It has been found in the present invention that a microorganism having an ability to produce 3-hydroxyadipic acid and/or α-hydromuconic acid, wherein the reaction to generate malic acid from oxaloacetic acid, and the reaction to generate acetyl-CoA from pyruvic acid are enhanced, can produce 3-hydroxyadipic acid and/or α-hydromuconic acid in high yields.

Hereinafter, 3-hydroxyadipic acid may be abbreviated as 3HA, and α-hydromuconic acid may be abbreviated as HMA. In addition, 3-oxoadipyl-CoA may be abbreviated as 3OA-CoA, 3-hydroxyadipyl-CoA may be abbreviated as 3HA-CoA, and 2,3-dehydroadipyl-CoA may be abbreviated as HMA-CoA. In addition, phosphoenolpyruvate may be abbreviated as PEP. In addition, the enzyme that catalyzes the reaction where 3-oxoadipyl-CoA is reduced to generate 3-hydroxyadipyl-CoA may be referred to as "3-oxoadipyl-CoA reductase." In addition, the complex of the proteins encoded

by the aceE, aceF and lpd genes in the presence of a single promoter may be referred to as pyruvate dehydrogenase complex and abbreviated as PDHc. In addition, the aceE, accF and lpd genes may be collectively referred to as PDHc gene cluster. In addition, pyruvate formate-lyase and the pyruvate formate-lyase activating enzyme may be collectively referred to as pyruvate formate-lyase, and abbreviated as PFL or Pfl. In addition, malate dehydrogenase may be abbreviated as MDH. In addition, formate dehydrogenase may be abbreviated as FDH. In addition, a nucleic acid encoding a functional polypeptide may be referred to as a gene.

The genetically modified microorganism of the present invention can biosynthesize 3-hydroxyadipic acid and/or α-hydromuconic acid via acetyl-CoA and succinyl-CoA as intermediates, as shown in the metabolic pathway described below. The metabolic pathway from glucose up to acetyl-CoA is well-known as glycolytic pathway, and the metabolic pathway up to succinyl-CoA as TCA cycle.

[Chem 1]

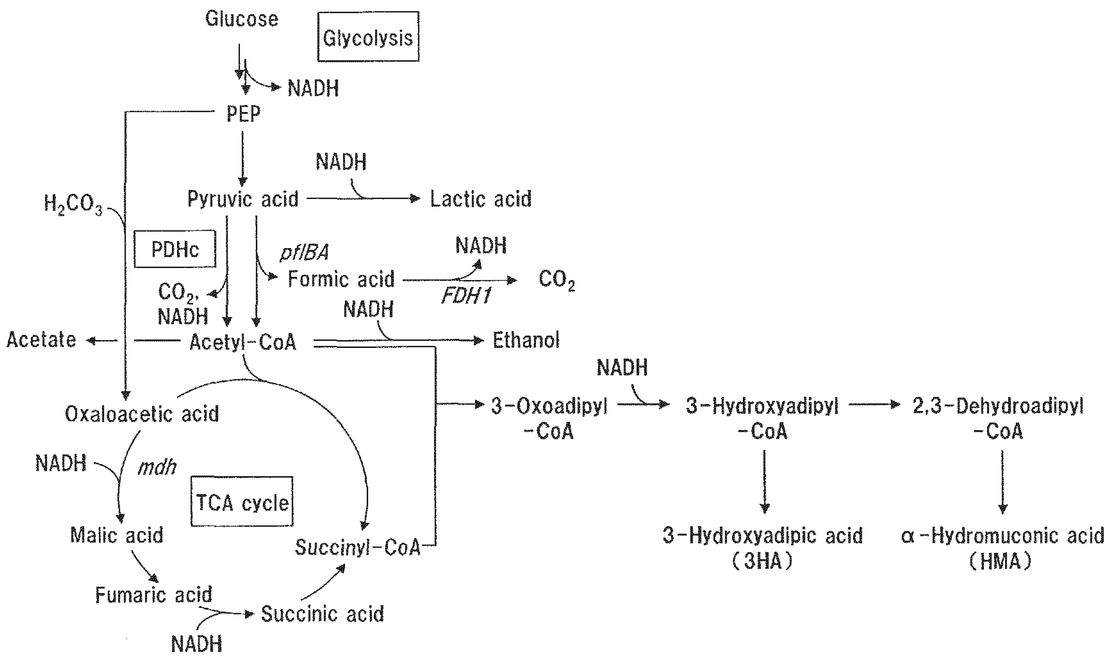


The metabolic pathways to produce 3-hydroxyadipic acid and/or α-hydromuconic acid from acetyl-CoA obtained in the glycolytic pathway and succinyl-CoA obtained in the TCA cycle are shown below, with the metabolites represented by the chemical formulae. In this scheme, the reaction A represents a reaction to generate 3-oxoadipyl-CoA from acetyl-CoA and succinyl-CoA. The reaction B represents a reaction that reduce 3-oxoadipyl-CoA to generate 3-hydroxyadipyl-CoA. The reaction C represents a reaction to generate 2,3-dehydroadipyl-CoA from 3-hydroxyadipyl-CoA. The reaction D represents a reaction to generate 3-hydroxyadipic acid from 3-hydroxyadipyl-CoA. The reaction E represents a reaction to generate α-hydromuconic acid from 2,3-dehydroadipyl-CoA. The enzymes that catalyze the reactions in the following metabolic pathway, and the method of creating a microorganism having an ability to produce 3-hydroxyadipic acid and/or α-hydromuconic acid using the following metabolic pathway are described in detail in WO 2019/107516. It is particularly preferred in the present invention that the reactions A, B, D, and E be enhanced by introducing a plasmid comprising genes encoding enzymes that catalyze the reactions A, B, D, and E. The methods themselves of enhancing these reactions are known, and described in detail in WO 2019/107516, and also described specifically in the following Examples.

[Chem 2]

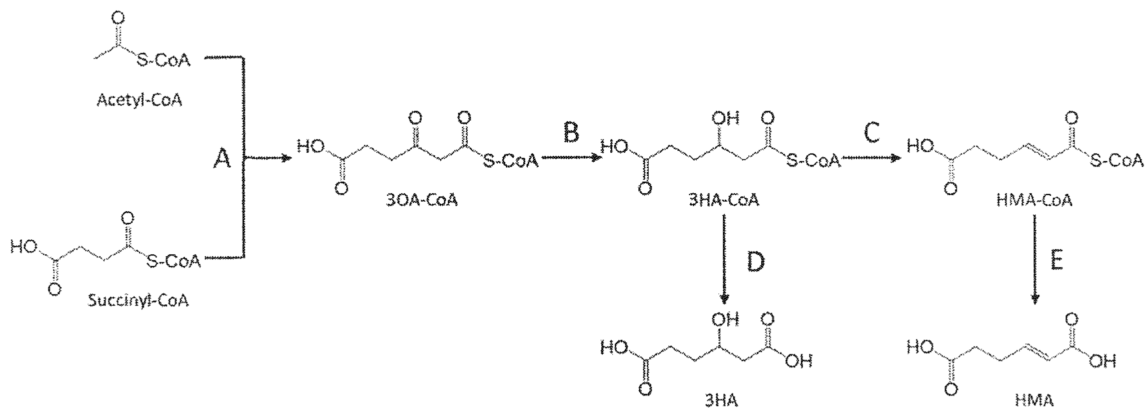

Methods to enhance the reaction to generate malic acid from oxaloacetic acid in the present invention include enhancing the reaction catalyzed by malate dehydrogenase. Malate dehydrogenase to be enhanced is not particularly limited, provided that it has a catalytic activity for the production of malic acid from oxaloacetic acid. For example, in bacteria such as of the genera *Escherichia* and *Serratia,* malate dehydrogenase (EC1.1.1.37) is encoded by the mdh gene. Malate dehydrogenase catalyzes the reaction that generates malic acid and NAD+ from oxaloacetic acid and NADH.

Specific examples of malate dehydrogenase include mdh derived from *Escherichia coli* str. K-12 substr. MG1655 (NCBI-Protein ID: NP_417703 (SEQ ID NO: 41)), and mdh derived from *Serratia grimesii* strain NBRC13537 (SEQ ID NO: 1). Whether or not the polypeptide encoded by a gene possessed by the microorganism used in the present invention is malate dehydrogenase can be determined by performing BLAST search on the public database in NCBI, KEGG, or the like.

The method of enhancing the reaction catalyzed by malate dehydrogenase may be, for example, enhancing the catalytic activity of malate dehydrogenase itself, or increasing the expression of malate dehydrogenase, and preferably is increasing the expression of malate dehydrogenase. Examples of the method of increasing the expression of malate dehydrogenase include methods in which the malate dehydrogenase gene is introduced into a host microorganism from outside the microorganism; in which the copy number of the gene is increased; and in which the promoter region or the ribosome binding sequence upstream of the coding region of the gene is modified. These methods may be carried out individually or in combination. Of these, the method is preferable in which a plasmid comprising the gene encoding malate dehydrogenase is introduced into a host microorganism (see the following Examples). The amino acid sequence of malate dehydrogenase is known as described above (such as SEQ ID NOs: 1 and 41), and known genes encoding the known amino acid sequences can be introduced. It is noted that any polypeptide having the malate dehydrogenase activity may be used, in which a known amino acid sequence, for example, represented by SEQ ID NO: 1 or 41 has a mutation(s) such as replacement, deletion, and/or insertion. In this case, preferred are those having a sequence identity of preferably 95% or more, more preferably 97% or more, still more preferably 99% or more to a known wild-type amino acid sequence. As used herein, the sequence identity means the number of matching amino acids when sequences are aligned such that the highest number of matching amino acids is obtained, divided by the total number of amino acids (the number of the longer amino acids when the total numbers of amino acids are different), expressed as a percentage, which can be easily calculated using well-known software such as FASTA (the same applies hereinafter).

As described later, Escherichia microorganisms are those having an ability to produce 3-hydroxyadipic acid and $\alpha$-hydromuconic acid. Biotechnol Lett. 2011 Dec;33(12):2439-44 has reported that *Escherichia coli* with deletion of the pflB gene encoding pyruvate formate-lyase that catalyzes the reaction to generate acetyl-CoA from pyruvic acid, wherein the expression of the mdh gene encoding malate dehydrogenase catalyzing a reaction to generate malic acid from oxaloacetic acid is enhanced, shows increased yield of succinic acid. On the other hand, in the present invention, the reaction to generate acetyl-CoA from pyruvic acid and the reaction to generate malic acid from oxaloacetic acid are enhanced. Thus, it is difficult to expect based on the description in the literature that, in production of 3-hydroxyadipic acid and/or $\alpha$-hydromuconic acid, simultaneous enhancement of the reaction to generate acetyl-CoA from pyruvic acid and the reaction to generate malic acid from oxaloacetic acid has an effect to increase the production of 3-hydroxyadipic acid and/or $\alpha$-hydromuconic acid. However, in the present invention, microorganisms having an ability to produce 3-hydroxyadipic acid and/or $\alpha$-hydromuconic acid, in which the reaction to generate malic acid from oxaloacetic acid is enhanced, and the reaction to generate acetyl-CoA from pyruvic acid is enhanced, unexpectedly show increased yield(s) of 3-hydroxyadipic acid and/or $\alpha$-hydromuconic acid.

One method to enhance the reaction to generate acetyl-CoA from pyruvic acid in the present invention is a method of enhancing the reaction catalyzed by the pyruvate dehydrogenase complex (PDHc). The PDHc to be enhanced

is not particularly limited, provided that it has a catalytic activity for the production of acetyl-CoA from pyruvic acid. For example, in bacteria such as those belonging to the genus *Escherichia* or the genus *Serratia,* the PDHc is composed of pyruvate dehydrogenase (E1, EC1.2.4.1), dihydrolipoyl transacetylase (E2, EC2.3.1.12), and dihydrolipoyl dehydrogenase (E3, ECl .8.1.4), which are encoded by aceE, aceF, and lpd genes, respectively. PDHc catalyzes, as a whole, the reactions that generate acetyl-CoA, $CO_2$, and NADH from pyruvic acid, coenzyme A, and NAD+. The PDHc gene cluster form an opcron, and the expression of the PDHc gene cluster is regulated by a PDHc transcriptional repressor (PdhR) encoded by the pdhR gene. The transcription of the PDHc gene cluster is repressed in the presence of PdhR, so that the expression of PDHc is decreased. On the other hand, the transcription of the PDHc gene cluster is not inhibited in the absence of PdhR, so that the expression of PDHc is increased.

Specific examples of PDHc include AceE (NCBI-Protein ID: NP_414656), AceF (NCBI-Protein ID: NP_414657), and Lpd (NCBI-Protein ID: NP_414658) derived from *Escherichia coli* str. K-12 substr. MG1655; AceE (SEQ ID NO: 2), AceF (SEQ ID NO: 3), and Lpd (SEQ ID NO: 4) derived from *Serratia grimesii* strain NBRC13537; and LpdA (NCBI-Protein ID: ABR75580) derived from *Klebsiella pneumoniae* subsp. pneumoniae strain MGH 78578. Whether or not the polypeptide encoded by a gene possessed by the microorganism used in the present invention is PDHc can be determined by performing BLAST search on the public database in NCBI (National Center for Biotechnology Information), KEGG (Kyoto Encyclopedia of Genes and Genomes), or the like.

Specific examples of the PDHc transcriptional repressor include PdhR derived from *Escherichia coli* str. K-12 substr. MG1655 (NCBI-Protein ID: NP_414655 (SEQ ID NO:42)), and PdhR derived from *Serratia grimesii* strain NBRC13537 (SEQ ID NO: 5). Whether or not the polypeptide encoded by a gene possessed by the microorganism used in the present invention is a PDHc transcriptional repressor can be determined by performing BLAST search on the public database in NCBI (National Center for Biotechnology Information), KEGG (Kyoto Encyclopedia of Genes and Genomes), or the like. Deletion of such PDHc transcriptional repressor is preferable (see the following Examples). As described above, PDHc transcriptional repressor and the gene encoding it are known, and thus the deletion of PDHc transcriptional repressor can be easily achieved by routine procedures. For example, the deletion can be easily achieved by cutting out and removing the gene encoding PDHc transcriptional repressor, or inserting an arbitrary nucleotide sequence into or deleting an arbitrary nucleotide sequence from the gene to cause a frameshift mutation.

Methods of enhancing the reaction catalyzed by PDHc include, for example, increasing the expression of at least one enzyme that constitutes PDHc. Methods of increasing the expression of at least one enzyme that constitutes PDHc include, for example, introducing at least one gene that constitutes the PDHc gene cluster into host microorganisms from outside the microorganisms; increasing the copy numbers of the gene cluster; and modifying the promoter regions or the ribosome-binding sequences upstream of the coding regions of the gene cluster. These methods may be carried out individually or in combination. The increase in the expression of PDHc can also be achieved by decreasing the function of the PDHc transcriptional repressor.

Other methods of enhancing the reaction catalyzed by PDHc include, for example, enhancing the activity of PDHc. To enhance the activity of PDHc, the catalytic activity of at least one enzyme constituting PDHc may be enhanced. Specific methods of enhancing the PDHc activity include, for example, reducing the sensitivity to NADH. Reduced sensitivity to NADH means, for example, that the KI value of the enzyme for NADH is two or more times higher than the control. PDHc with reduced sensitivity for NADH is obtained by using a E354K and/or H322Y variant of Lpd derived from *Escherichia coli* str. K-12 (NCBI-Protein ID: NP_414658 (SEQ ID NO: 43)) described in Kim et al., J. Bacteriol. 190: 3851-3858 (2008), or LpdA derived from *Klebsiella pneumoniae* subsp. pneumoniae strain MGH 78578 (NCBI-Protein ID: ABR75580 (SEQ ID NO: 44)), which is known to function under anaerobic environments, or a part thereof. The enzyme gene with enhanced catalytic activity may be replaced by or coexist with the wild-type gene originally contained in the microorganism used in the production. Alternatively, the copy number of the enzyme gene may be increased, or the promoter region or ribosome-binding sequence upstream of the coding region of the enzyme gene may be modified. These methods may be carried out individually or in combination. Of these, the method is preferable in which a plasmid comprising the gene encoding the above-described PDHc with reduced sensitivity to NADH is introduced into a host microorganism (see the following Examples). The amino acid sequence of PDHc with reduced sensitivity to NADH is known as described above (such as SEQ ID NOS: 43 and 44), and known genes encoding the known amino acid sequences can be introduced. It is noted that any polypeptide having the PDHc activity may be used, in which a known amino acid sequence, for example, represented by SEQ ID NO: 43 or 44 has mutation such as replacement, deletion, or insertion. In this case, preferred are those having a sequence identity of preferably 95% or more, more preferably 97% or more, still more preferably 99% or more to a known wild-type amino acid sequence.

One method to enhance the reaction to generate acetyl-CoA from pyruvic acid in the present invention include enhancing the reaction catalyzed by pyruvate formate-lyase. The pyruvate formate-lyase to be enhanced is not particularly limited, provided that it has a catalytic activity for production of acetyl-CoA from pyruvic acid. For example, in bacteria such as *Escherichia* and *Serratia,* pyruvate formate-lyase (EC2.3.1.54) functions in the presence of pyruvate formate-lyase activating enzyme (EC1.97.1.4), which are encoded by pflB and pflA genes, respectively. Pyruvate formate-lyase catalyzes the reactions that generate acetyl-CoA and formic acid from pyruvic acid and coenzyme A. pflB and pflA

form an operon.

Specific examples of pyruvate formate-lyase include PflB (NCBI-Protein ID: NP_415423 (SEQ ID NO: 45)) and PflA (NCBI-Protein ID: NP_415422 (SEQ ID NO: 46)) derived from *Escherichia coli* str. K-12 substr. MG1655, and PflB (SEQ ID NO: 6) and PflA (SEQ ID NO: 7) derived from *Serratia grimesii* strain NBRC13537. Whether or not the polypeptide encoded by a gene possessed by the microorganism used in the present invention is pyruvate formate-lyase can be determined by performing BLAST search on the public database in NCBI, KEGG, or the like.

The method of enhancing the reaction catalyzed by pyruvate formate-lyase may be, for example, enhancing the catalytic activity of pyruvate formate-lyase itself, or increasing the expression of pyruvate formate-lyase, and preferably is increasing the expression of pyruvate formate-lyase. Methods of increasing the expression of pyruvate formate-lyase include, for example, introducing the pyruvate formate-lyase gene into host microorganisms from outside the microorganisms; increasing the copy number of the gene; and modifying the promoter region or the ribosome binding sequence upstream of the coding region of the gene. These methods may be carried out individually or in combination. Of these, the method is preferable in which a plasmid comprising the gene encoding pyruvate formate-lyase is introduced into a host microorganism (see the following Examples). The amino acid sequence of pyruvate formate-lyase is known as described above (such as SEQ ID NOS: 45 and 46), and known genes encoding the known amino acid sequences can be introduced. It is noted that any polypeptide having the pyruvate formate-lyase activity may be used, in which a known amino acid sequence, for example, represented by SEQ ID NO: 45 or 46 has a mutation(s) such as replacement, deletion, and/or insertion. In this case, preferred are those having a sequence identity of preferably 95% or more, more preferably 97% or more, still more preferably 99% or more to a known wild-type amino acid sequence.

Incidentally, US 2017/0298363 A1 describes production of adipic acid by using a non-naturally occurring microorganism, in which the metabolism of the microorganism having the phosphoketolase pathway is modified to increase the energy efficiency. In this document, it is described that the glucose metabolism through the glycolytic pathway generates NADH, but the glucose metabolism through the phosphoketolasc pathway does not generate NADH, and thus, in the case of production of reduced compounds using a microorganism having the phosphoketolase pathway, the expression or activity of PDHc or pyruvate formate-lyase and an NAD(P)H-generating formate dehydrogenase is enhanced in order to improve the shortage of NADH in the metabolism of the microorganism. Here, adipic acid is a more reduced compound than 3-hydroxyadipic acid and $\alpha$-hydromuconic acid, and thus it is thought by those skilled in the art that enhancing the PDHc and/or pyruvate formate-lyase reaction(s) in production of 3-hydroxyadipic acid and/or $\alpha$-hydromuconic acid leads to oxidation-reduction imbalance, and the yield of the compound is decreased.

US 2017/0298363 A1 also describes malate dehydrogenase as one of pathways to improve the lack of NADH. Here, to obtain NADH using malate dehydrogenase, the reaction is required to proceed in the direction of generating oxaloacetic acid from malic acid. On the other hand, as is apparent from the metabolic pathway, the reaction is desired to proceed in the direction of generating malic acid from oxaloacetic acid to obtain 3-hydroxyadipic acid and/or $\alpha$-hydromuconic acid. Progression of the reaction in the opposite direction leads to a decrease in the supply of succinyl-CoA. Therefore, those skilled in the art would consider that enhancement of the malate dehydrogenase reaction for production of 3-hydroxyadipic acid and/or $\alpha$-hydromuconic acid also results in reduced yield(s) of 3-hydroxyadipic acid and/or $\alpha$-hydromuconic acid due to reduced supply of succinyl-CoA.

However, the present invention gives an unexpected effect that culturing of a genetically modified microorganism having an ability to produce 3-hydroxyadipic acid and/or $\alpha$-hydromuconic acid, in which the reaction to generate acetyl-CoA from pyruvic acid is enhanced, and the reaction catalyzed by malate dehydrogenase is enhanced, results in improved production of 3-hydroxyadipic acid and/or $\alpha$-hydromuconic acid.

It is also preferred in the present invention that the reaction to reduce 3-oxoadipyl-CoA to generate 3-hydroxyadipyl-CoA be enhanced. Enhancing the reaction that reduces 3-oxoadipyl-CoA to generate 3-hydroxyadipyl-CoA includes, for example, enhancing the activity of the enzyme that catalyzes the reaction. Examples of the method to enhance the activity of the enzyme include introducing the enzyme gene into host microorganisms from outside the microorganisms; increasing the copy number of the gene; and modifying the promoter region or the ribosome binding sequence upstream of the coding region of the gene. These methods may be performed solely or in combination, but it is preferred that the enzyme gene be introduced into a host microorganism from outside the microorganism by the method described in WO 2019/107516 (US 2020/291435 A1).

Specific examples of the 3-oxoadipyl-CoA reductase that catalyzes the reaction where 3-oxoadipyl-CoA is reduced to generate 3-hydroxyadipyl-CoA include enzymes classified into EC1 .1.1.35 as 3-hydroxyacyl-CoA dehydrogenases; enzymes classified into EC1.1.1.157 as 3-hydroxybutyryl-CoA dehydrogenases; and enzymes having 70% or more sequence identity with any amino acid sequence of SEQ ID NOs: 1 to 6 and 213 and having a 3-oxoadipyl-CoA reductase activity disclosed in WO 2019/107516 (US 2020/291435 A1). Specific examples of the enzyme that catalyzes the reaction where 3-oxoadipyl-CoA is reduced to generate 3-hydroxyadipyl-CoA include PaaH derived from *Pseudomonas putida* strain KT2440 (NCBI-Protein ID: NP 745425. 1), PaaH derived from *Escherichia coli* str. K-12 substr. MG1655 (NCBI-Protein ID: NP_415913.1), DcaH derived from *Acinetobacter baylyi* strain ADP1 (NCBI-Protein ID: CAG68533.1), PaaH derived from *Serratia plymuthica* strain NBRC102599 (NCBI-Protein ID: WP_063197120), and a

polypeptide derived from *Serratia marcescens* strain ATCC 13880 (NCBI-Protein ID: KFD11732.1).

It is preferred that the genetically modified microorganism of the present invention be a microorganism that does not undergo glucose metabolism via the phosphoketolase pathway from the viewpoint of productivity of 3-hydroxyadipic acid and/or $\alpha$-hydromuconic acid. Such a microorganism exists in the nature and can be preferably used as a host for creation of the genetically microorganism of the present invention that does not undergo glucose metabolism via the phosphoketolase pathway. A microorganism that does not undergo glucose metabolism via the phosphoketolase pathway may also be created by a method comprising creating the genetically modified microorganism of the present invention and then knocking out the phosphoketolase gene of the microorganism by a method well known by those skilled in the art.

The genetically modified microorganism of the present invention preferably has enhanced reaction to generate carbon dioxide from formic acid in addition to the modification described above. Preferably, the method to enhance the reaction to generate carbon dioxide from formic acid is to enhance the enzymatic reaction having catalytic activity of generating carbon dioxide from formic acid. Examples of the method include enhancing the catalytic activity of the enzyme itself, and increasing the expression of the enzyme, and increasing the expression the enzyme is preferred. Examples of the method to enhance the expression of an enzyme having catalytic activity of generating carbon dioxide from formic acid include introducing the gene encoding the enzyme into a host microorganism from outside the microorganism; increasing the copy number of the gene; and modifying the promoter region or the ribosome binding sequence upstream of the coding region of the gene. The method of introducing a gene encoding an enzyme having catalytic activity of generating carbon dioxide from formic acid into a host microorganism from outside the microorganism, or of increasing the copy number of the gene may be a method in which the gene originally possessed by the host gene is artificially introduced, or a method in which the gene that is exogenous is introduced. As a method to increase the expression of an enzyme having catalytic activity of generating carbon dioxide from formic acid, the methods described above may be performed individually or in combination.

Specific examples of the enzyme having catalytic activity to generate carbon dioxide from formic acid include enhancement of the reaction catalyzed by NADH-generating formate dehydrogenase (EC 1.17.1.9). NADH-generating formate dehydrogenase catalyzes the reaction in which carbon dioxide is generated from formic acid, and NAD+ is reduced into NADH. Specific examples of NADH-generating formate dehydrogenase are the polypeptides found as NADH-generating formate dehydrogenase by BLAST search on public database such as in NCBI or KEGG. Preferred specific examples include FDH1 derived from *Candida boidinii* strain S2 (NCBI-Protein ID: AAC49766.1 (SEQ ID NO: 47)), and homologs thereof. A method is preferable in which a plasmid comprising the gene encoding such NADH-generating formate dehydrogenase is introduced into a host microorganism (see the following Examples). The amino acid sequence of NADH-generating formate dehydrogenase is known as described above (such as SEQ ID NO: 47), and known genes (such as SEQ ID NO: 34) encoding the known amino acid sequences can be introduced. It is noted that any polypeptide having the NADH-generating formate dehydrogenase activity may be used, in which a known amino acid sequence, for example, represented by SEQ ID NO: 47 has a mutation(s) such as replacement, deletion, and/or insertion. In this case, preferred are those having a sequence identity of preferably 95% or more, more preferably 97% or more, still more preferably 99% or more to a known wild-type amino acid sequence.

Other specific examples of the enzyme having catalytic activity to generate carbon dioxide from formic acid include formate hydrogenlyase complex. Formate hydrogenlyase complex catalyzes the reaction to generate hydrogen during generation of carbon dioxide from formic acid. Preferred specific examples of formate hydrogenlyase complex include formate dehydrogenase H encoded by the fdhF gene (formate dehydrogenase-H, EC 1.17.98.4), and formate hydrogenlyase complex composed of seven polypeptide subunits encoded by the hycB, hycC, hycD, hycE, hycF, and hycG genes (formate hydrogenlyase complex, EC 1.17.98.4), in bacteria such as *Escherichia.*

Other specific examples of the enzyme having catalytic activity to generate carbon dioxide from formic acid also include formate dehydrogenase O and/or formate dehydrogenase N. Formate dehydrogenase O and/or formate dehydrogenase N catalyze(s) the reaction to allow quinones to be reduced during generation of carbon dioxide from formic acid.

Preferred specific examples of formate dehydrogenase O and/or formate dehydrogenase N include formate dehydrogenase O composed of three polypeptide subunits encoded by the fdoG, fdoH, and fdoI genes (formate dehydrogenase-O, EC 1.17.5.3), and formate dehydrogenase N composed of three polypeptide subunits encoded by the fdnG, fdnH, and fdnI genes (formate dehydrogenase-N, EC 1.17.5.3), in bacteria such as *Escherichia.*

In the present invention, among the enzymes listed above, enhancement of the reaction catalyzed by NADH-generating formate dehydrogenase is preferred.

Microorganisms originally having an ability to produce 3-hydroxyadipic acid include the following microorganisms:

the genus *Escherichia,* such as *Escherichia fergusonii* and *Escherichia coli*;
the genus *Serratia,* such as *Serratia grimesii, Serratia ficaria, Serratia fonticoia, Serratia odorifera, Serratia plymuthica, Serratia enlomophila,* and *Serratia nematodiphila;*

- the genus *Pseudomonas,* such as *Pseudomonas chlororaphis, Pseudomonas putida, Pseudomonas azotoformans,* and *Pseudomonas chlororaphis* subsp. aureofaciens;
- the genus *Hafnia,* such as *Hafnia alvei;*
- the genus *Corynebacterium,* such as *Corynebacterium* acetoacidophilum, *Corynebacterium acetoglutamicum, Corynebacterium ammoniagenes,* and *Corynebacterium glutamicum*;
- the genus *Bacillus,* such as *Bacillus badius, Bacillus megaterium,* and *Bacillus roseus;*
- the genus *Streptomyces,* such as *Streptomyces vinaceus, Streptomyces karnatakensis,* and *Streptomyces olivaceus;*
- the genus *Cupriavidus,* such as *Cupriavidus metallidurans, Cupriavidus necator,* and *Cupriavidus oxalaticus*;
- the genus *Acinetobacter,* such as *Acinetobacter baylyi,* and *Acinetobacter radioreslstens;*
- the genus *Alcaligenes,* such as *Alcaligenes faecalis;*
- the genus *Nocardioides,* such as *Nocardioides albus;*
- the genus *Brevibacterium,* such as *Brevibacterium iodinum;*
- the genus *Delftia,* such as *Delftia acidovorans;*
- the genus *Shimwellia,* such as *Shimwellia blattae;*
- the genus *Aerobacter,* such as *Aerobacter cloacae;* and
- the genus *Rhizobium,* such as *Rhizobium radiobacter.*

Among the microorganisms originally having an ability to produce 3-hydroxyadipic acid, the microorganisms belonging to the genus *Escherichia, Serratia, Hafnia,* or *Corynebacterium,* such as *Corynebacterium acetoacidophllum, Corynebacterium acetoglutamicum, Corynebacterium ammoniagenes,* and *Corynebacterium glutamicum, Brevibacterium, Shimwellia,* or *Aerobacter,* which are microorganisms that do not undergo glucose metabolism via the phosphoketolase pathway, are preferably used in the present invention, and microorganisms belonging to the genus *Escherichia* or *Serratia* are more preferably used.

Microorganisms that are speculated to originally have the ability to produce α-hydromuconic acid include the following microorganisms:

- the genus *Escherichia,* such as *Escherichiafergusonii* and *Escherichia coli;*
- the genus *Serratia,* such as *Serratia grimesii, Serrαtia ficaria, Serratia, fonticola, Serratia odorifera, Serratia plymuthica, Serratia entomophila,* and *Serratia nematodiphila:,*
- the genus *Pseudomonas,* such as *Pseudomonas fluorescens, Pseudomonas putida, Pseudomonas azotoformans,* and *Pseudomonas chlororaphis* subsp. aureofaciens;
- the genus *Hafnia,* such as *Hafnia alvei;*
- the genus *Bacillus,* such as *Bacillus badius;*
- the genus *Cupriavidus,* such as *Cupriavidus metallidurans, Cupriavidus numazuensis,* and *Cupriavidus oxalaticus*;
- the genus *Acinetobacter,* such as *Acinetobacter baylyi* and *Acinetobacter radioresistens;*
- the genus *Alcaligenes,* such as *Alcaligenes faecalis*;
- the genus *Delftia,* such as *Delftia acidovorans;* and
- the genus *Shimwellia,* such as *Shimwellia blattae*;

Among the microorganisms originally having an ability to produce α-hydromuconic acid, the microorganisms belonging to the genus *Escherichia, Serratia, Hafnia,* or *Shimwellia,* which are microorganisms that do not undergo glucose metabolism via the phosphoketolase pathway, are preferably used in the present invention, and a microorganism belonging to the genus *Escherichia* or *Serratia* is more preferably used.

When the genetically modified microorganism of the present invention does not originally have the ability to produce 3-hydroxyadipic acid, an appropriate combination of nucleic acids encoding the enzymes that catalyze the reactions A, B, and D may be introduced into the microorganisms to impart the ability to produce them. On the other hand, when the genetically modified microorganism of the present invention does not originally have the ability to produce α-hydromuconic acid, an appropriate combination of nucleic acids encoding the enzymes that catalyze the reactions A, B, C, and E may be introduced into the microorganism to impart the ability to produce them.

In the present invention, the microorganisms that can be used as hosts to obtain the genetically modified microorganisms are not limited to particular microorganisms, provided that they can be genetically modified, and may be microorganisms with or without the ability to produce 3-hydroxyadipic acid and/or α-hydromuconic acid, and are preferably microorganisms belonging to the genus *Escherichia, Serratia, Hafnia, Pseudomonas, Corynebacterium, Bacillus, Streptomyces, Cupriavidus, Acinetobacter, Alcaligenes, Brevibacterium, Delftia, Shimwellia, Aerobacter, Rhizobium, Thermobifida, Clostridium, Schizosaccharomyces, Kluyveromyces, Pichia,* or *Candida,* more preferably microorganisms belonging to the genus *Escherichia, Serratia, Hafnia,* or *Pseudomonas,* and particularly preferably microorganisms belonging to the genus *Escherichia* or *Serratia.*

The method to introduce a gene to create the genetically modified microorganism of the present invention is not limited to a particular method, and examples of the method that can be used include a method in which the gene incorporated in an expression vector capable of autonomous replication in a microorganism is introduced into a host microorganism, and a method in which the gene is integrated into the genome of a microorganism.

The gene(s) to be introduced may be a single gene or a plurality of genes. Moreover, introduction of a gene(s) and enhancement of expression may be combined.

When a gene expressed in the present invention is integrated into an expression vector or the genome of a host microorganism, the nucleic acid which is integrated into the expression vector or the genome is preferably composed of a promoter, a ribosome-binding sequence, a gene to be expressed, and a transcription termination sequence. In addition, the nucleic acid may also contain a gene that controls the activity of the promoter.

The promoter used in the present invention is not limited to a particular promoter, provided that the promoter drives expression of the gene in the host microorganism; examples of the promoter include gap promoter, trp promoter, lac promoter, tac promoter, and T7 promoter.

In cases where an expression vector is used in the present invention to introduce genes or to enhance the expression of genes, the expression vector is not limited to a particular vector, provided that the vector is capable of autonomous replication in the microorganism; examples of the vector include pBBR1MCS vector, pBR322 vector, pMW vector, pET vector, pRSF vector, pCDF vector, pACYC vector, and derivatives of the above vectors.

In cases where a nucleic acid for genome integration is used in the present invention to introduce the gene or to enhance the expression of the gene, the nucleic acid for genome integration is introduced by site-specific homologous recombination. The method for site-specific homologous recombination is not limited to a particular method, and examples of the method include a method in which λ Red recombinase and FLP recombinase are used (Proc Natl Acad Sci U.S.A. 2000 Jun 6; 97 (12): 6640-6645.), and a method in which λ Red recombinase and the sacB gene are used (Biosci Biotechnol Biochem. 2007 Dec; 71 (12): 2905-11.).

The method of introducing the expression vector or the nucleic acid for genome integration is not limited to a particular method, provided that the method is for introduction of a nucleic acid into a microorganism; examples of the method include the calcium ion method (Journal of Molecular Biology, 53,159 (1970)), and electroporation (NM Calvin, PC Hanawalt. J. Bacteriol, 170 (1988), pp. 2796-2801).

The genetically modified microorganism of the present invention is cultured in a culture medium, preferably a liquid culture medium, containing a carbon source as a material for fermentation which can be used by ordinary microorganisms. The culture medium used contains, in addition to the carbon source that can be used by the genetically modified microorganism, appropriate amounts of a nitrogen source, inorganic salts, and, if necessary, organic trace nutrients such as amino acids and vitamins. Any of natural and synthetic culture medium can be used as long as the medium contains the above-described nutrients.

The material for fermentation is a material that can be metabolized by the genetically modified microorganism. The term "metabolize" refers to conversion of a chemical compound, which a microorganism has taken up from the extracellular environment or intracellularly generated from a different chemical compound, to another chemical compound through an enzymatic reaction. Sugars can be suitably used as the carbon source. Specific examples of the sugars include monosaccharides, such as glucose, sucrose, fructose, galactose, mannose, xylose, and arabinose; disaccharides and polysaccharides formed by linking these monosaccharides; and saccharified starch solution, molasses, and saccharified solution from cellulose-containing biomass, each containing any of those saccharides.

The above-listed carbon sources may be used individually or in combination, and culturing in a culture medium containing glucose is particularly preferred. When a carbon source is added, the concentration of the carbon source in the culture medium is not particularly limited, and can be appropriately selected depending on the type of the carbon source, or the like. A preferred concentration of glucose is from 5 to 300 g/L.

As the nitrogen source used for culturing the genetically modified microorganism, for example, ammonia gas, aqueous ammonia, ammonium salts, urea, nitric acid salts, other supportively used organic nitrogen sources, such as oil cakes, soybean hydrolysate, casein degradation products, other amino acids, vitamins, corn steep liquor, yeast or yeast extract, meat extract, peptides such as peptone, and bacterial cells and hydrolysate of various fermentative bacteria can be used. The concentration of the nitrogen source in the culture medium is not particularly limited, and is preferably from 0.1 to 50 g/L.

As the inorganic salts used for culturing the genetically modified microorganism, for example, phosphoric acid salts, magnesium salts, calcium salts, iron salts, and manganese salts can be appropriately added to the culture medium and used.

The culture conditions for the genetically modified microorganism to produce 3-hydroxyadipic acid and/or α-hydromuconic acid are set by appropriately adjusting or selecting, for example, the culture medium with the above composition, culture temperature, stirring speed, pH, aeration rate, and inoculation amount, depending on the species of the genetically modified microorganism and external conditions, and the like.

The pH range in the culturing is not particularly limited, provided that the genetically modified microorganism can

grow, and is preferably from pH5 to 8, more preferably from pH5.5 to 6.8.

The range of the aeration rate condition in the culturing is not particularly limited, provided that 3-hydroxyadipic acid and/or α-hydromuconic acid can be produced, and it is preferred that oxygen remain in the gas and/or liquid phase in the culture vessel at least at the start of the culturing in order to allow for good growth of the microbial mutant.

In cases where foam is formed in a liquid culture, an antifoaming agent such as a mineral oil, silicone oil, or surfactant may be appropriately added to the culture medium.

After a recoverable amount of 3-hydroxyadipic acid and/or α-hydromuconic acid is produced during culturing of the microorganism, the produced products can be recovered. The produced products can be recovered, for example isolated, according to a commonly used method, in which the culturing is stopped once a product of interest is accumulated to an appropriate level, and the fermentation product is collected from the culture. Specifically, the products can be isolated from the culture by separation of bacterial cells through, for example, centrifugation or filtration prior to, for example, column chromatography, ion exchange chromatography, activated charcoal treatment, crystallization, membrane separation, or distillation. More specifically, examples include, but are not limited to, a method in which an acidic component is added to salts of the products, and the resulting precipitate is collected; a method in which water is removed from the culture by concentration using, for example, a reverse osmosis membrane or an evaporator to increase the concentrations of the products and the products and/or salts of the products are then crystallized and precipitated by cooling or adiabatic crystallization to recover the crystals of the products and/or salts of the products by, for example, centrifugation or filtration; and a method in which an alcohol is added to the culture to produce esters of the products and the resulting esters of the products are subsequently collected by distillation and then hydrolyzed to recover the products. These recovery methods can be appropriately selected and optimized depending on, for example, physical properties of the products.

Adipic acid can be produced by allowing the 3-hydroxyadipic acid and/or α-hydromuconic acid according to the present invention to react with hydrogen (be hydrogenated) in the presence of a hydrogenation catalyst.

The hydrogenation catalyst preferably contains a transition metal element, specifically preferably one or two or more selected from the group consisting of palladium, platinum, ruthenium, rhodium, rhenium, nickel, cobalt, iron, iridium, osmium, copper, and chromium, and more preferably one or two or more selected from the group consisting of palladium, platinum, nickel, cobalt, iron, copper, and chromium.

The hydrogenation catalyst is preferably used with being supported on a support from the viewpoints, for example, that the amount of metal used can be saved and the active surface of the catalyst is increased. Supporting the hydrogenation catalyst on support can be carried out according to a known method, such as impregnation, deposition-precipitation, and vapor deposition. Examples of the support include carbon, polymer, metallic oxide, metallic sulfide, zeolite, clay, heteropoly acid, solid phosphoric acid, and hydroxyapatite.

Hydrogen to be reacted with 3-hydroxyadipic acid and/or α-hydromuconic acid may be added simultaneously or successively. The partial pressure of hydrogen is not limited, but too low partial pressure of hydrogen results in longer reaction time, while too high partial pressure of hydrogen is undesirable for equipment safety. Thus, the partial pressure of hydrogen, at normal temperature, is preferably from 0.1 MPa to 10 MPa (gauge pressure), more preferably from 0.3 MPa to 5 MPa (gauge pressure), and still more preferably from 0.5 MPa to 3 MPa (gauge pressure).

The reaction manner may be one using any of the following reactors: batch tank reactors, semi-batch tank reactors, continuous stirred-tank reactors, continuous plug flow reactors, and trickle-bed tubular reactors. When using a solid hydrogenation catalyst, the reaction can be performed in any of the following manners: suspension, immobilized-bed, moving-bed, and fluidized-bed manners.

The reaction temperature for hydrogenation is not particularly limited, but too low reaction temperatures result in slow reaction rate, and too high reaction temperatures result in high energy consumption, which are thus undesirable. From such a point of view, the reaction temperature is preferably from 100 to 350°C, more preferably from 120 to 300°C, still more preferably from 130 to 280°C, still more preferably from 140 to 250°C, still more preferably from 150 to 230°C, and still more preferably from 160 to 220°C.

The atmosphere in the reactor may contain inert gas, such as nitrogen, helium, and argon, in addition to hydrogen. However, the oxygen concentration is preferably 5 vol% or less because oxygen causes deterioration of hydrogenation catalyst and generation of detonating gas. In addition, from the viewpoint of the stability of 3-hydroxyadipic acid and/or α-hydromuconic acid, and of adipic acid, the amount of ammonia relative to 3-hydroxyadipic acid and/or α-hydromuconic acid is preferably 5 wt% or less, more preferably 3 wt% or less, and still more preferably 0 wt% (i.e., the reaction in the absence of ammonia).

Preferably, 3-hydroxyadipic acid and/or α-hydromuconic acid is/are hydrogenated in the presence of a solvent.

Examples of the solvent that can be used for hydrogenation include methanol, ethanol, n-propanol, isopropanol, n-butanol, isobutanol, tert-butanol, pentane, hexane, cyclohexane, heptane, octane, decane, dimethyl ether, diethyl ether, 1,2-dimethoxyethane, diglyme, tetrahydrofuran, dioxane, methyl acetate, ethyl acetate, n-propyl acetate, n-butyl acetate, γ-butyrolactone, N-methylpyrrolidone, dimethyl sulfoxide, and aqueous solvents. A mixed solvent containing two or more of them may be used, but preferably an aqueous solvent is used from the viewpoints of economy and environmental friendliness.

In the present invention, aqueous solvents mean water or mixed solvents mainly containing water mixed with water-miscible organic solvents. The term "mainly containing water" means that the percentage of water in a mixed solvent is more than 50 vol%, preferably 70 vol% or more, and still more preferably 90 vol% or more.

Examples of the water-miscible organic solvent include methanol, ethanol, n-propanol, isopropanol, n-butanol, isobutanol, tert-butanol, 1,2-dimethoxyethane, diglyme, tetrahydrofuran, dioxane, γ-butyrolactone, N-methylpyrrolidone, dimethyl sulfoxide, dimethylformamide, dimethylacetamide, and acetone.

The pH of the aqueous solvent is not particularly limited, but is preferably pH2 to 13, more preferably pH3 to 11, and still more preferably pH4 to 10, in consideration of for example, prevention of catalyst deterioration, prevention of by-product production, and corrosiveness to reaction equipment.

The charge amount of 3-hydroxyadipic acid and/or α-hydromuconic acid relative to the solvent is not particularly limited, but low charge amount is not desirable from an industrial standpoint. From such a point of view, the charge amount of 3-hydroxyadipic acid and/or α-hydromuconic acid relative to 100 parts by weight of the solvent is, in terms of an amount of 3-hydroxyadipic acid-3,6-lactone, preferably from 0.1 parts by weight to 900 parts by weight, more preferably from 0.2 parts by weight to 800 parts by weight, and still more preferably from 1.0 part by weight to 700 parts by weight.

In the case where a solvent other than primary alcohols or secondary alcohols is used as the solvent for hydrogenation, a corresponding adipic acid, adipic acid salt, or adipic acid ester is generated from the carboxylic acid, carboxylic acid salt, carboxylic acid ester of 3-hydroxyadipic acid and/or α-hydromuconic acid. In the case where a solvent containing a primary alcohol or secondary alcohol, such as methanol, ethanol, n-propanol, isopropanol, n-butanol, or isobutanol, is used as the solvent for hydrogenation, a mixture of adipic acid, adipic acid salt, adipic acid monoester, and adipic acid diester is obtained after the reaction. Carboxylic acid, a carboxylic acid salt, a carboxylic acid ester of adipic acid, and a mixture thereof are collectively referred to as "adipic acid" in the present description.

Carboxylic acid of adipic acid obtained in the present invention can be further subjected to esterification reaction to be converted into an adipic acid ester. The esterification method is not particularly limited, and include, for example, dehydration condensation between carboxylic acid and alcohol using an acid catalyst and a condensing agent, and methods using alkylating agents such as diazomethane and alkyl halides.

Adipic acid obtained in the present invention can be isolated and purified by common unit operations such as centrifugation, filtration, membrane filtration, distillation, extraction, crystallization, and dehydration.

Adiponitrile can be produced from adipic acid obtained in the present invention by a known method (e.g., JP 61-24555 B). The obtained adiponitrile can be hydrogenated by a known method (e.g., JP 2000-508305 A) to produce hexamethylenediamine.

Adipic acid obtained in the present invention can be polycondensed with diamine according to a known method (see, for example, "Polyamide Resin Handbook," Nikkan Kogyo Shimbun, edited by Osamu Fukumoto, January 1998) to produce polyamide. Specifically, 1,4-diaminobutane, 1,5-pentanediamine, or hexamethylenediamine as diamine can be used to produce polyamide 46, polyamide 56, or polyamide 66, respectively.

Polyamide can be processed according to known methods (e.g., WO 2019/208427) to produce polyamide fibers. The thus-obtained polyamide fibers can be used for clothing applications such as innerwear, sportwear, and casual wear, as well as industrial material applications such as airbags and tire cords.

Polyamide can be molded into polyamide molded products according to known methods (e.g., WO 2021/006257). The thus-obtained polyamide molded products can be used for automotive parts, electrical parts, electronic parts, construction parts, various containers, daily necessities, household goods, and sanitary goods.

## EXAMPLES

The present invention will now be specifically described by way of Examples. It is noted that the various analyses in the Examples followed the following methods.

### Quantitative analyses of substrate and product

The supernatant of the culture medium from which bacterial cells were removed by centrifugation was processed by membrane treatment using Millex-GV (0.22 μm; PVDF; manufactured by Merck), and the resulting filtrate was analyzed according to the following method to measure the concentrations of 3-hydroxyadipic acid and α-hydromuconic acid accumulated in the culture supernatant, and of sugars remaining in the culture medium without being used. Additionally, based on the results, the yields of 3-hydroxyadipic acid and α-hydromuconic acid were calculated using the following Formula (1).

Yield (%) = amount of formed product (mol) / amount of consumed sugar (mol) × 100

Formula (1)

Quantitative analyses of 3-hydroxyadipic acid and α-hydromuconic acid by LC-MS/MS HPLC: 1290 Infinity (manufactured by Agilent Technologies, Inc.)
Column: Synergi hydro-RP (manufactured by Phenomenex Inc.), length: 100 mm, internal diameter: 3 mm, particle size: 2.5 μm
Mobile phase: 0.1% aqueous formic acid solution / methanol = 70/30
Flow rate: 0.3 mL/min
Column temperature: 40°C
LC detector: 1260DAD VL+ (210 nm)
·MS/MS: Triple-Quad LC/MS (manufactured by Agilent Technologies, Inc.)
Ionization method: ESI in negative mode

Quantitative analyses of sugars by HPLC
HPLC: Shimazu Prominence (manufactured by Shimadzu Corporation)
Column: Shodex Sugar SH1011 (manufactured by Showa Denko K.K.), length: 300 mm, internal diameter: 8 mm, particle size: 6 μm
Mobile phase: 0.05 M aqueous sulfuric acid solution
Flow rate: 0.6 mL/min
Column temperature: 65°C
Detector: RID-10A (manufactured by Shimadzu Corporation)

Reference Example 1

Production of a plasmid expressing an enzyme catalyzing a reaction to generate 3OA-CoA and coenzyme A from acetyl-CoA and succinyl-CoA (the reaction A) and an enzyme catalyzing a reaction to generate 3HA-CoA from 3OA-CoA (the reaction B), and a reaction to generate 3-hydroxyadipic acid from 3HA-CoA (the reaction D) and a reaction to generate α-hydromuconic acid from HMA-CoA (the reaction E)

The pBBR1MCS-2 vector (ME Kovach, (1995), Gene 166: 175-176), capable of autonomous replication in *E. coli,* was cleaved with XhoI to obtain pBBR1MCS-2/XhoI. To integrate a constitutive expression promoter into the vector, primers (SEQ ID NOs: 9 and 10) were designed for use in amplification of an upstream 200-b region (SEQ ID NO: 8) of gapA (NCBI Gene ID: NC_000913.3) by PCR using the genomic DNA of *Escherichia coli* str. K-12 substr. MG1655 as a template, and a PCR reaction was performed in accordance with routine procedures. The resulting fragment and the pBBR1MCS-2/XhoI were ligated together using the In-Fusion HD Cloning Kit (manufactured by Takara Bio Inc.), and the resulting plasmid was introduced into E. *coli* strain DH5α. The nucleotide sequence on the plasmid extracted from the obtained recombinant *E. coli* strain was confirmed in accordance with routine procedures, and the plasmid was designated as pBBRIMCS-2::Pgap. Then, the pBBR1MCS-2::Pgap was cleaved with Seal to obtain pBBR1MCS-2::Pgap/ScaI. To amplify a gene encoding an enzyme catalyzing the reaction A, primers (SEQ ID NOs: 11 and 12) were designed for use in amplification of the full length of the acyltransferase gene pcaF (NCBI Gene ID: 1041755) by PCR using the genomic DNA of *Pseudomonas putida* strain KT2440 as a template, and a PCR reaction was performed in accordance with routine procedures. The resulting fragment and the pBBR1MCS-2::Pgap/ScaI were ligated together using the In-Fusion HD Cloning Kit, and the resulting plasmid was introduced into *E. coli* strain DH5α. The nucleotide sequence on the plasmid isolated from the obtained recombinant strain was confirmed in accordance with routine procedures, and the plasmid was designated as pBBR1MCS-2::AT. Then, the pBBR1MCS-2::AT was cleaved with HpaI to obtain pBBR1MCS-2::AT/HpaI. To amplify a gene encoding an enzyme catalyzing the reactions D and E, primers (SEQ ID NOs: 13 and 14) were designed for use in amplification of a continuous sequence including the full lengths of genes together encoding a CoA transferase, pcaI and pcaJ (NCBI Gene IDs: 1046613 and 1046612), by PCR using the genomic DNA of *Pseudomonas putida* strain KT2440 as a template, and a PCR reaction was performed in accordance with routine procedures. The resulting fragment and the pBBR1MCS-2::AT/HpaI were ligated together using the In-Fusion HD Cloning Kit, and the resulting plasmid was introduced into *E. coli* strain DH5α. The nucleotide sequence on the plasmid isolated from the obtained recombinant strain was confirmed in accordance with routine procedures, and the plasmid was designated as pBBR1MCS-2::ATCT.

The pBBR1MCS-2::ATCT was cleaved with ScaI to obtain pBBR1MCS-2::ATCT/ScaI. Primers (SEQ ID NOs: 16 and 17) were designed for use in amplification of a nucleic acid represented by SEQ ID NO: 15 using the genomic DNA of *Serratia marcescens* strain ATCC 13880 as a template, and a PCR reaction was performed in accordance with routine procedures. The resulting fragment and the pBBR1MCS-2::ATCT/ScaI were ligated together using the In-Fusion HD Cloning Kit (manufactured by Takara Bio Inc.), and the resulting plasmid was introduced into *E. coli* strain DH5α. The nucleotide sequence on the plasmid isolated from the obtained recombinant strain was confirmed in accordance with routine procedures, and the plasmid was designated as pBBR1MCS-2::ATCTOR.

Reference Example 2

Preparation of mutant *Escherichia* microorganism with introduced plasmid for expression of enzymes that catalyze reactions A, B, D and E

The plasmids prepared in Reference Example 1 were introduced into *Escherichia coli* str. K-12 substr. MG1655 to prepare mutant *Escherichia* microorganisms.

*Escherichia coli* str. K-12 substr. MG1655 was seeded in 5 mL of LB medium and incubated with shaking at 30°C for 1 day. Subsequently, 0.5 mL each of the cultures was seeded in 5 mL of LB medium, and incubated at 30°C for 2 hours with shaking. The cultures were cooled on ice for 20 minutes, and then the bacterial cells were washed with 10%(w/w) glycerol 3 times. The washed pellet was resuspended in 100 μL of 10%(w/w) glycerol, mixed with 1 μL of pBBR1MCS-2::ATCTOR, and then cooled in an electroporation cuvette on ice for 10 minutes. Electroporation was performed (3 kV, 200 Ω, 25 μF) using Gene pulser (manufactured by Bio-Rad Laboratories, Inc.), immediately after which 1 mL of SOC culture medium was added and incubated at 30°C for 1 hour with shaking. Subsequently, 50 μL each of the cultures was applied to LB agar medium containing 25 μg/mL kanamycin and incubated at 30°C for 1 day. The resulting strain was designated as Ec/3IIA.

Reference Example 3

Preparation of mutant *Escherichia* microorganism without increased expression of Mdh and with introduced plasmid expressing enzymes that catalyze reactions A, B, D and E

Subsequently, Ec/3HA was seeded in 5 mL of LB medium containing 25 μg/mL kanamycin, and incubated at 30°C for 1 hour with shaking. Subsequently, 0.5 mL each of the cultures was seeded in 5 mL of LB medium containing 25 μg/mL kanamycin, and incubated at 30°C for 2 hours with shaking. The cultures were cooled on ice for 20 minutes, and then the bacterial cells were washed with 10%(w/w) glycerol 3 times. The washed pellet was resuspended in 100 μL of 10%(w/w) glycerol, mixed with 1 μL of pMW119, and then cooled in an electroporation cuvette on ice for 10 minutes. Electroporation was performed (3 kV, 200 Ω, 25 μF) using Gene pulser (manufactured by Bio-Rad Laboratories, Inc.), immediately after which 1 mL of SOC culture medium was added and incubated at 30°C for 1 hour with shaking. Subsequently, 50 μL each of the cultures was applied to LB agar medium containing 25 μg/mL kanamycin and 100 μg/mL ampicillin, and incubated at 30°C for 1 day. The resulting strain was designated as Ec/3HApMW.

Reference Example 4

Test for production of 3-hydroxyadipic acid and α-hydromuconic acid using mutant *Escherichia* microorganism without increased expression of Mdh and with introduced plasmid expressing enzymes that catalyze reactions A, B, D and E

The mutants prepared in Reference Example 3 were used to perform a test for production of 3-hydroxyadipic acid and α-hydromuconic acid.

A loopful of each mutant prepared in Reference Example 3 was inoculated into 5 mL (φ18-mm glass test tube, aluminum plug) of the culture medium I (10 g/L Bacto Tryptone (manufactured by Difco Laboratories), 5 g/L Bacto Yeast Extract (manufactured by Difco Laboratories), 5 g/L sodium chloride, 25 μg/mL kanamycin, 100 μg/mL ampicillin) adjusted to pH 7, and incubated at 30°C for 24 hours with shaking at 120 $min^{-1}$. Subsequently, 0.25-1 mL of the culture fluid was added to 5 mL (φ18-mm glass test tube, aluminum plug) of the culture medium II (50 g/L glucose, 1 g/L ammonium sulfate, 50 mM potassium phosphate, 0.025 g/L magnesium sulfate, 0.0625 mg/L iron sulfate, 2.7 mg/L manganese sulfate, 0.33 mg/L calcium chloride, 1.25 g/L sodium chloride, 2.5 g/L Bacto Tryptone, 1.25 g/L Bacto Yeast Extract, 25 μg/mL kanamycin, 100 μg/mL ampicillin) adjusted to pH 6.5, and incubated at 30°C with shaking. The concentrations of 3-hydroxyadipic acid and α-hydromuconic acid accumulated in the culture supernatant, and of sugars remaining in the culture medium without being used were measured, and the yields of 3-hydroxyadipic acid and α-hydromuconic acid calculated are presented in Table 1.

Reference Example 5

Preparation of plasmid for expression of Mdh

The pCDF-1b expression vector (manufactured by Merck Millipore), which is capable of autonomous replication in *E. coli,* was cleaved with BamHI to obtain pCDF/BamHI. To integrate a constitutive expression promoter into the vector, primers (SEQ ID NOs: 18 and 19) were designed for use in amplification of an upstream 200-b region (SEQ ID NO: 8) of gapA (NCBI Gene ID: NC_000913.3) by PCR using the genomic DNA of *Escherichia coli* str. K-12 substr. MG1655 as a template, and a PCR reaction was performed in accordance with routine procedures. The resulting fragment and pCDF/BamHI were ligated together using the In-Fusion HD Cloning Kit (manufactured by Takara Bio Inc.), and the resulting plasmid was introduced into *E. coli* strain DH5α. The nucleotide sequence on the plasmid extracted from the obtained recombinant *E. coli* strain was confirmed in accordance with routine procedures, and the plasmid was designated as pCDF::Pgap. Then, the pCDF::Pgap was cleaved with BamHI to obtain pCDF::Pgap/BamHI. To amplify a gene encoding malate dehydrogenase, primers (SEQ ID NOs: 20 and 21) were designed for use in amplification of the region comprising the full length of mdh (NCBI Gene ID: 947854) by PCR using the genomic DNA of *Escherichia coli* str. K-12 substr. MG1655 as a template, and a PCR reaction was performed in accordance with routine procedures. The resulting fragment and pCDF::Pgap/BamHI were ligated together using the In-Fusion HD Cloning Kit (manufactured by Takara Bio Inc.), and the resulting plasmid was introduced into *E. coli* strain DH5α. The nucleotide sequence on the plasmid extracted from the obtained streptomycin-resistant strain was confirmed in accordance with routine procedures. The obtained plasmid was designated as pCDF::mdh.

Next, to integrate the sequence of a region comprising the gapA promoter and mdh of pCDF::mdh into an expression vector pMW119 that is capable of autonomous replication in *E. coli* (manufactured by Nippon Gene Co., Ltd.), pCDF::mdh and pMW119 were cleaved with XbaI and SacI, and the resulting fragments were ligated together to obtain a plasmid pMW119::mdh. The nucleotide sequence on the plasmid was confirmed in accordance with routine procedures.

Comparative Example 1

Preparation of mutant *Escherichia* microorganism with increased expression of Mdh and with introduced plasmid expressing enzymes that catalyze reactions A, B, D and E

The plasmid pMW1 19::mdh prepared in Reference Example 5 was introduced into Ec/3HA in the same manner as in Reference Example 3 to prepare a mutant *Escherichia* microorganism. The resulting strain was designated as Ec/3HAmdh.

Comparative Example 2

Test for production of 3-hydroxyadipic acid and α-hydromuconic acid using mutant *Escherichia* microorganism with increased expression of Mdh and with introduced plasmid expressing enzymes that catalyze reactions A, B, D and E

Ec/3HAmdh prepared in Comparative Example 1 was cultured in the same manner as in Reference Example 4. The concentrations of 3-hydroxyadipic acid and α-hydromuconic acid accumulated in the culture supernatant, and of sugars remaining in the culture medium without being used were measured, and the yields of 3-hydroxyadipic acid and α-hydromuconic acid calculated are as presented in Table 1. It was demonstrated that the mutant *Escherichia* microorganisms with increased expression of mdh and with an introduced plasmid expressing enzymes that catalyze the reactions A, B, D and E showed reduced yields of 3-hydroxyadipic acid and α-hydromuconic acid.

Reference Example 6

Preparation of mutant *Escherichia* microorganism with reduced function of pyruvate dehydrogenase complex transcriptional repressor

pdhR, which is a gene (NCBI Gene ID: 944827) coding for the pyruvate dehydrogenase complex transcriptional repressor (NCBI-ProteinID:NP_414655, SEQ ID NO: 42) of *Escherichia* microorganisms *(Escherichia coli* str. K-12 substr. MG1655), was deleted to prepare a mutant *Escherichia* microorganism with increased expression of PDHc.

PCR was performed using pKD4 as a template and oligo DNAs represented by SEQ ID NOs: 22 and 23 as primers to obtain a PCR fragment for pdhR deficiency. pKD46 was introduced into *Escherichia coli* str. K-12 substr. MG1655, and then the PCR fragment for pdhR deficiency was introduced. The resulting kanamycin-resistant strain was subjected to colony direct PCR to confirm that the gene of interest was deleted and the kanamycin resistance gene was

inserted based on the band lengths. The primers used were oligo DNAs represented by SEQ ID NOs: 24 and 26.

Thereafter, pKD46 was allowed to be lost to obtain ampicillin-sensitive strains. pCP20 was introduced into the ampicillin-sensitive strains to again obtain ampicillin-resistant strains. The resulting strains were subjected to colony direct PCR to confirm that the kanamycin resistance gene was lost based on the band lengths. The primers used were oligo DNAs represented by SEQ ID NOs: 25 and 26. The kanamycin-sensitive strains were allowed to lose pCP20. The resulting strain was designated as EcΔR.

Reference Example 7

Preparation of mutant *Escherichia* microorganism with reduced function of pyruvate dehydrogenase complex transcriptional repressor and with introduced plasmid expressing enzymes that catalyze reactions A, B, D and E

The plasmid prepared in Reference Example 1 was introduced into *Escherichia* microorganism strain EcΔR in the same manner as in Reference Example 2 to prepare a mutant *Escherichia* microorganism. The resulting strain was designated as EcΔR/3HA.

Comparative Example 3

Preparation of mutant *Escherichia* microorganism without increased expression of Mdh, and with reduced function of pyruvate dehydrogenase complex transcriptional repressor, and with introduced plasmid expressing enzymes that catalyze reactions A, B, D and E

A plasmid pMW119 was introduced into EcΔR/3HA prepared in Reference Example 7, in the same manner as in Comparative Example 1. The resulting strain was designated as EcΔR/3HApMW.

Comparative Example 4

Test for production of 3-hydroxyadipic acid and α-hydromuconic acid using mutant *Escherichia* microorganism without increased expression of Mdh, and with reduced function of pyruvate dehydrogenase complex transcriptional repressor and with introduced plasmid expressing enzymes that catalyze reactions A, B, D and E

The mutant *Escherichia* microorganisms prepared in Comparative Example 3 were cultured in the same manner as in Reference Example 3 except that the culture medium additionally contained 100 μg/mL ampicillin. The concentrations of 3-hydroxyadipic acid and α-hydromuconic acid accumulated in the culture supernatant, and of sugars remaining in the culture medium without being used were measured, and the yield of 3-hydroxyadipic acid calculated is as presented in Table 1. It was demonstrated that the mutant *Escherichia* microorganisms without increased expression of Mdh, and with reduced function of pyruvate dehydrogenase complex transcriptional repressor, and with an introduced plasmid expressing enzymes that catalyze the reactions A, B, D and E showed increased yields of 3-hydroxyadipic acid and α-hydromuconic acid.

Example 1

Preparation of mutant *Escherichia* microorganism with increased expression of Mdh, and with reduced function of pyruvate dehydrogenase complex transcriptional repressor, and with introduced plasmid expressing enzymes that catalyze reactions A, B, D and E

pMW119::mdh was introduced into EcΔR/3HA prepared in Reference Example 6, in the same manner as in Comparative Example 1. The resulting strain was designated as EcΔR/3HAmdh.

Example 2

Test for production of 3-hydroxyadipic acid and α-hydromuconic acid using mutant *Escherichia* microorganism with increased expression of Mdh, and with reduced function of pyruvate dehydrogenase complex transcriptional repressor and with introduced plasmid expressing enzymes that catalyze reactions A, B, D and E

The mutant *Escherichia* microorganisms prepared in Example 1 were cultured in the same manner as in Comparative Example 4. The concentration of 3-hydroxyadipic acid accumulated in the culture supernatant, and of sugars remaining in the culture medium without being used were measured, and the yields of 3-hydroxyadipic acid and α-hydromuconic acid calculated is as presented in Table 1. It was demonstrated that the mutant *Escherichia* microorganisms with increased expression of Mdh, and with reduced function of pyruvate dehydrogenase complex transcriptional repressor, and with an introduced plasmid expressing enzymes that catalyze the reactions A, B, D and E showed further increased yields of 3-hydroxyadipic acid and $\alpha$-hydromuconic acid.

[Table 1]

| | strain | 3HA yield (%) | HMA yield (%) |
|---|---|---|---|
| Reference Example 4 | Ec/3HApMW | 5.8 | 0.023 |
| Comparative Example 2 | Ec/3HAmdh | 5.5 | 0.021 |
| Comparative Example 4 | Ec$\Delta$R/3HApMW | 8.2 | 0.026 |
| Example 2 | Ec$\Delta$R/3HAmdh | 9.5 | 0.028 |
| Example 10 | Ec$\Delta$R/3HAmdh_fdh | 10.1 | 0.028 |

Reference Example 8

Preparation of plasmid for expression of Lpd derived from *Escherichia coli* or LpdA derived from *Klebsiella pneumoniae*

The pMW119 expression vector (manufactured by Nippon Gene Co., Ltd.) capable of autonomous replication in *E. coli,* was cleaved with SacI and KpnI to obtain pMW119/SacI, KpnI.

To amplify a gene encoding Lpd derived from *Escherichia coli,* primers (SEQ ID NOs: 27 and 28) were designed for use in amplification of the region comprising the full lengths of lpd (NCBI Gene ID: 944854) and 0.5 kb on its 5' side and 0.1 kb on its 3' side by PCR using the genomic DNA of *Escherichia coli* str. K-12 substr. MG1655 as a template, and a PCR reaction was performed in accordance with routine procedures. The resulting fragment and pMW119/SacI, KpnI were ligated together using the In-Fusion HD Cloning Kit (manufactured by Takara Bio Inc.), and the resulting plasmid was introduced into *E. coli* strain DH5$\alpha$. The nucleotide sequence on the plasmid extracted from the obtained recombinant strain was confirmed in accordance with routine procedures. The obtained plasmid was designated as pMW119::EcLPD.

To amplify a gene encoding LpdA derived from *Klebsiella pneumoniae,* gene synthesis was performed for a region comprising the full length of lpdA of *Klebsiella pneumoniae* subsp. pneumoniae MGH78578 (NCBI Gene ID: CP000647, REGION: 142460..143884), and 0.5 kb on the 5' side and 0.5 kb on the 3' side of lpd of *Escherichia coli* str. K-12 substr. MG1655, followed by designing primers (SEQ ID NOs: 27 and 29) for use in amplification of the region by PCR, and then a PCR reaction was performed in accordance with routine procedures. The resulting fragment and pMW119/SacI, KpnI were ligated together using the In-Fusion HD Cloning Kit (manufactured by Takara Bio Inc.), and the resulting plasmid was introduced into *E. coli* strain DH5$\alpha$. The nucleotide sequence on the plasmid extracted from the obtained recombinant strain was confirmed in accordance with routine procedures. The obtained plasmid was designated as pMW119::KpLPD.

Reference Example 9

Preparation of plasmid for expression of PFL

To amplify a gene encoding PFL, primers (SEQ ID NOs: 30 and 31) were designed for use in amplification of the region comprising the full lengths of pflB (NCBI Gene ID: 945514) and pflA (NCBI Gene ID: 945517) by PCR using the genomic DNA of *Escherichia coli* str. K-12 substr. MG1655 as a template, and a PCR reaction was performed in accordance with routine procedures. The resulting fragment and pMW119::Pgap/SphI were ligated together using the In-Fusion HD Cloning Kit (manufactured by Takara Bio Inc.), and the resulting plasmid was introduced into *E. coli* strain DH5$\alpha$. The nucleotide sequence on the plasmid extracted from the obtained recombinant strain was confirmed in accordance with routine procedures. The obtained plasmid was designated as pMW119::PFL.

Reference Example 10

Preparation of plasmid for expression of Lpd with reduced sensitivity to NADH

An amino acid variant of pMW119::EcLPD obtained in Reference Example 8 was prepared. Substitution of glutamic acid (E) with lysine (K) at position 354 was performed using Q5 Site-Directed Mutagenesis Kit (manufactured by New England Biolabs, Inc.), pMW119::EcLPD as a template, and oligonucleotides represented by SEQ ID NOs: 32 and 33

as primers. The obtained plasmid was designated as pMW119::EcLPD_E354K.

Reference Example 11

Preparation of mutant *Escherichia* microorganism without increased expression of Mdh, Lpd, or PFL and with introduced plasmid expressing enzymes that catalyze reactions A, B, D and E

Subsequently, Ec/3HApMW was seeded in 5 mL of LB medium containing 25 μg/mL kanamycin and 100 μg/mL ampicillin, and incubated at 30°C for 1 hour with shaking. Subsequently, 0.5 mL each of the cultures was seeded in 5 mL of LB medium containing 25 μg/mL kanamycin and 100 μg/mL ampicillin, and incubated at 30°C for 2 hours with shaking. The cultures were cooled on ice for 20 minutes, and then the bacterial cells were washed with 10%(w/w) glycerol 3 times. The washed pellet was resuspended in 100 μL of 10%(w/w) glycerol, mixed with 1 μL of pMW119::Pgap, and then cooled in an electroporation cuvette on ice for 10 minutes. Electroporation was performed (3 kV, 200 Ω, 25 μF) using Gene pulser (manufactured by Bio-Rad Laboratories, Inc.), immediately after which 1 mL of SOC culture medium was added and incubated at 30°C for 1 hour with shaking. Subsequently, 50 μL each of the cultures was applied to LB agar medium containing 25 μg/mL kanamycin, 100 μg/mL ampicillin, and 50 μg/mL streptomycin, and incubated at 30°C for 1 day. The resulting strain was designated as Ec/3HApCDF_pMW.

Reference Example 12

Test for production of 3-hydroxyadipic acid and α-hydromuconic acid using mutant *Escherichia* microorganism without increased expression of Mdh, Lpd, or PFL, and with introduced plasmid expressing enzymes that catalyze reactions A, B, D and E

Ec/3HApCDF_pMW prepared in Reference Example 11 was cultured in the same manner as in Example 4 except that the culture medium contained 50 μg/mL streptomycin. The concentrations of 3-hydroxyadipic acid and α-hydromuconic acid accumulated in the culture supernatant, and of sugars remaining in the culture medium without being used were measured, and the yields of 3-hydroxyadipic acid and α-hydromuconic acid calculated are presented in Table 2.

Comparative Example 5

Preparation of mutant *Escherichia* microorganism with increased expression of Mdh, and without increased expression of Lpd or PFL, and with introduced plasmid expressing enzymes that catalyze reactions A, B, D and E

pCDF::mdh prepared in Reference Example 5 was introduced into Ec/3HApMW, in the same manner as in Reference Example 11. The resulting strain was designated as Ec/3HAmdh_pMW.

Comparative Example 6

Test for production of 3-hydroxyadipic acid and α-hydromuconic acid using mutant *Escherichia* microorganism with increased expression of Mdh, and without increased expression of Lpd or PFL, and with introduced plasmid expressing enzymes that catalyze reactions A, B, D and E

Ec/3HAmdh_pMW prepared in Comparative Example 5 was cultured in the same manner as in Reference Example 12. The concentrations of 3-hydroxyadipic acid and α-hydromuconic acid accumulated in the culture supernatant, and of sugars remaining in the culture medium without being used were measured, and the yields of 3-hydroxyadipic acid and α-hydromuconic acid calculated are as presented in Table 2. It was demonstrated that the mutant *Escherichia* microorganisms with increased expression of mdh and with an introduced plasmid expressing enzymes that catalyze the reactions A, B, D and E, even transduced with an empty vector of pMW119, showed reduced yield of 3-hydroxyadipic acid, but the yield of α-hydromuconic acid did not change.

Example 3

Preparation of mutant *Escherichia* microorganism with increased expression of Mdh and Lpd, and with introduced plasmid expressing enzymes that catalyze reactions A, B, D and E

The plasmids prepared in Reference Examples 5 and 8 were introduced into Ec/3HA prepared in Reference Example 2 to prepare mutant *Escherichia* microorganisms.

Subsequently, Ec/3HA was seeded in 5 mL of LB medium containing 25 μg/mL kanamycin, and incubated at 30°C for 1 hour with shaking. Subsequently, 0.5 mL each of the cultures was seeded in 5 mL of LB medium containing 25 μg/mL kanamycin, and incubated at 30°C for 2 hours with shaking. The cultures were cooled on ice for 20 minutes, and then the bacterial cells were washed with 10%(w/w) glycerol 3 times. The washed pellet was resuspended in 100 μL of 10%(w/w) glycerol, mixed with 1 μL of pCDF::mdh and 1 μL of pMW119::KpLPD, and then cooled in an electroporation cuvette on ice for 10 minutes. Electroporation was performed (3 kV, 200 Ω, 25 μF) using Gene pulser (manufactured by Bio-Rad Laboratories, Inc.), immediately after which 1 mL of SOC culture medium was added and incubated at 30°C for 1 hour with shaking. Subsequently, 50 μL each of the cultures was applied to LB agar medium containing 25 μg/mL kanamycin, 100 μg/mL ampicillin, and 50 μg/mL streptomycin, and incubated at 30°C for 1 day. The resulting strain was designated as Ec/3HAmdh_KpLPD.

Example 4

Preparation of mutant *Escherichia* microorganism with increased expression of Mdh and PFL, and with introduced plasmid expressing enzymes that catalyze reactions A, B, D and E

The plasmids prepared in Reference Examples 5 and 9 were introduced into Ec/3HA prepared in Reference Example 2 to prepare mutant *Escherichia* microorganisms. pCDF::mdh and pMW119::PFL prepared in Reference Examples 5 and 9 were introduced into Ec/3HA, in the same manner as in Example 3. The resulting strain was designated as Ec/3HAmdh_PPL.

Example 5

Preparation of mutant *Escherichia* microorganism with increased expression of Mdh, and after introduction of plasmid for expression of Lpd with reduced sensitivity to NADH and enzymes that catalyze reactions A, B, D and E

pCDF::mdh and pMW119::EcLPD_E354K prepared in Reference Examples 5 and 10 were introduced into Ec/3HA, in the same manner as in Example 3. The resulting strain was designated as Ec/3HAmdh_LPD _E354K.

Example 6

Test for production of 3-hydroxyadipic acid and α-hydromuconic acid using mutant *Escherichia* microorganism with increased expression of Mdh and Lpd, and with introduced plasmid expressing enzymes that catalyze reactions A, B, D and E

Ec/3HAmdh_KpLPD prepared in Example 3 was cultured in the same manner as in Reference Example 12. The concentrations of 3-hydroxyadipic acid and α-hydromuconic acid accumulated in the culture supernatant, and of sugars remaining in the culture medium without being used were measured, and the yields of 3-hydroxyadipic acid and α-hydromuconic acid calculated are as presented in Table 2. It was demonstrated that the mutant *Escherichia* microorganisms with increased expression of Mdh and Lpd, and with an introduced plasmid expressing enzymes that catalyze the reactions A, B, D and E showed increased yields of 3-hydroxyadipic acid and α-hydromuconic acid.

Example 7

Test for production of 3-hydroxyadipic acid and α-hydromuconic acid using mutant *Escherichia* microorganism with increased expression of Mdh and PFL, and with introduced plasmid expressing enzymes that catalyze reactions A, B, D and E

Ec/3HAmdh_PFL prepared in Example 4 was cultured in the same manner as in Reference Example 12. The concentrations of 3-hydroxyadipic acid and α-hydromuconic acid accumulated in the culture supernatant, and of sugars remaining in the culture medium without being used were measured, and the yields of 3-hydroxyadipic acid and α-hydromuconic acid calculated are as presented in Table 2. It was demonstrated that the mutant *Escherichia* microorganisms with increased expression of Mdh and PFL, and with an introduced plasmid expressing enzymes that catalyze the reactions A, B, D and E showed increased yields of 3-hydroxyadipic acid and α-hydromuconic acid.

Example 8

Test for production of 3-hydroxyadipic acid and α-hydromuconic acid using mutant *Escherichia* microorganism with increased expression of Mdh, and after introduction of plasmid for expression of Lpd with reduced sensitivity to NADH and enzymes that catalyze reactions A, B, D and E

Ec/3HAmdh_LPD_E354K prepared in Example 5 was cultured in the same manner as in Reference Example 12. The concentrations of 3-hydroxyadipic acid and α-hydromuconic acid accumulated in the culture supernatant, and of sugars remaining in the culture medium without being used were measured, and the yields of 3-hydroxyadipic acid and α-hydromuconic acid calculated are as presented in Table 2. It was demonstrated that the mutant *Escherichia* microorganisms with increased expression of Mdh, and after introduction of plasmid for expression of Lpd with reduced sensitivity to NADH and enzymes that catalyze reactions A, B, D and E showed increased yields of 3-hydroxyadipic acid and α-hydromuconic acid.

Reference Example 13

Preparation of plasmid for co-expression of MDH and FDH1

A nucleotide sequence encoding FDHl was introduced into the MDII-expressing plasmids pCDF::mdh and pMW119::mdh prepared in Reference Example 5 to prepare expression plasmids, pCDF::FDHl-mdh and pMW119::FDH1-mdh, which are designed to co-express MDH and FDH1 under control of gapA promoter.
pCDF::mdh prepared in Reference Example 5 was cleaved with BsaI and KpnI to obtain pCDF::mdh/BsaI_KpnI. A nucleotide sequence (SEQ ID NO: 34) encoding a part of the gapA promoter and NADH-generating formate dehydrogenase (FDII1) derived from *Candida boidinii* strain S2, and a sequence (SEQ ID NO: 35) comprising a part of the gapA promoter and a part of mdh (NCBI-GeneID:947854) derived from *Escherichia coli* str. K-12 substr. MG1655 were genetically synthesized, followed by designing primers (SEQ ID NOs: 36 and 37) for use in amplification of the regions by PCR, and then a PCR reaction was performed in accordance with routine procedures. The resulting fragments and pCDF::mdh/BsaI_KpnI were ligated together using the "In-Fusion HD Cloning Kit" (manufactured by Takara Bio Inc.), and the resulting plasmid was introduced into *E. coli* strain DH5α. The nucleotide sequence on the plasmid isolated from the obtained streptomycin-resistant strain was confirmed in accordance with routine procedures, and the plasmid was designated as pCDF::FDHl -mdh.
Thereafter, pMW119::mdh prepared in Reference Example 5 was cleaved with SacI and NaeI to obtain pMW119::mdh/SacI_NaeI. A nucleotide sequence (SEQ ID NO: 34) encoding a part of the gapA promoter and NADH-generating formate dehydrogenase (FDH1) derived from *Candida boidinii* strain S2, and a sequence (SEQ ID NO: 38) comprising a part of the gapA promoter and a part of mdh (NCBI-GeneID:947854) derived from *Escherichia coli* str. K-12 substr. MG1655 were genetically synthesized, followed by designing primers (SEQ ID NOs: 39 and 40) for use in amplification of the regions by PCR, and then a PCR reaction was performed in accordance with routine procedures. The resulting fragments and pMW119::mdh/BsaI_KpnI were ligated together using the "In-Fusion HD Cloning Kit" (manufactured by Takara Bio Inc.), and the resulting plasmid was introduced into E. *coli* strain DH5α. The nucleotide sequence on the plasmid isolated from the obtained ampicillin-resistant strain was confirmed in accordance with routine procedures, and the plasmid was designated as pMW119::FDH1-mdh.

Example 9

Preparation of mutant *Escherichia* microorganism with increased expression of FDH1 and Mdh, and with reduced function of pyruvate dehydrogenase complex transcriptional repressor, and with introduced plasmid expressing enzymes that catalyze reactions A, B, D and E

pMW119::FDH1-mdh prepared in Reference Example 13 was introduced into EcΔR/3HA, in the same manner as in Comparative Example 1. The resulting strain was designated as EcΔR/3HAmdh_fdh.

Example 10

Test for production of 3-hydroxyadipic acid and α-hydromuconic acid using mutant *Escherichia* microorganism with increased expression of FDH1 and Mdh, and with reduced function of pyruvate dehydrogenase complex transcriptional repressor and with introduced plasmid expressing enzymes that catalyze reactions A, B, D and E

The mutant *Escherichia* microorganisms prepared in Example 9 were cultured in the same manner as in

Comparative Example 4. The concentration of 3-hydroxyadipic acid accumulated in the culture supernatant, and of sugars remaining in the culture medium without being used were measured, and the yields of 3-hydroxyadipic acid and α-hydromuconic acid calculated is as presented in Table 1. It was demonstrated that the mutant *Escherichia* microorganisms with increased expression of FDH1 and Mdh, and with reduced function of pyruvate dehydrogenase complex transcriptional repressor, and with an introduced plasmid expressing enzymes that catalyze the reactions A, B, D and E showed further increased yield of 3-hydroxyadipic acid.

Example 11

Preparation of mutant *Escherichia* microorganism with increased expression of FDH1, Mdh, and Lpd, and with introduced plasmid expressing enzymes that catalyze reactions A, B, D and E

pCDF::FDH1-mdh and pMWI 19::KpLPD prepared in Reference Examples 13 and 8 were introduced into Ec/3HA, in the same manner as in Example 3. The resulting strain was designated as Ec/3HAmdh_fdh_KpLPD.

Example 12

Preparation of mutant *Escherichia* microorganism with increased expression of FDH1, Mdh, and PFL, and with introduced plasmid expressing enzymes that catalyze reactions A, B, D and E

pCDF::PDH1-mdh and pMWI 19::PFE prepared Reference Examples 13 and 9 were introduced into Ec/3HA, in the same manner as in Example 3. The resulting strain was designated as Ec/3HAmdh_fdh_PFL.

Example 13

Preparation of mutant *Escherichia* microorganism with increased expression of FDH1, Mdh, and PFL, and with reduced function of pyruvate dehydrogenase complex transcriptional repressor, and with introduced plasmid expressing enzymes that catalyze reactions A, B, D and E

The plasmids prepared in Reference Examples 13 and 9 were introduced into EcAR/3HA prepared in Reference Example 7 to prepare mutant *Escherichia* microorganisms.

pCDF::FDH1-mdh and pMW119::PFL prepared Reference Examples 13 and 9 were introduced into EcΔR/3HA, in the same manner as in Example 3. The resulting strain was designated as EcΔR/3HAmdh_fdh_PFL.

Example 14

Preparation of mutant *Escherichia* microorganism with increased expression of FDH1 and Mdh, and after introduction of plasmid for expression of Lpd with reduced sensitivity to NADH and enzymes that catalyze reactions A, B, D and E

pCDF::FDH1-mdh and pMW119::EcLPD_E354K prepared in Reference Examples 13 and 10 were introduced into Ec/3HA, in the same manner as in Example 3. The resulting strain was designated as Ec/3HAmdh_fdh_LPD_E354K.

Example 15

Test for production of 3-hydroxyadipic acid and α-hydromuconic acid using mutant *Escherichia* microorganism with increased expression of FDH1, Mdh, and Lpd, and with introduced plasmid expressing enzymes that catalyze reactions A, B, D and E

Ec/3HAmdh_fdh_KpLPD prepared in Example 11 was cultured in the same manner as in Reference Example 12. The concentrations of 3-hydroxyadipic acid and α-hydromuconic acid accumulated in the culture supernatant, and of sugars remaining in the culture medium without being used were measured, and the yields of 3-hydroxyadipic acid and α-hydromuconic acid calculated are as presented in Table 2. It was demonstrated that the mutant *Escherichia* microorganisms with increased expression of FDH1, Mdh, and Lpd, and with an introduced plasmid expressing enzymes that catalyze the reactions A, B, D and E showed further increased yields of 3-hydroxyadipic acid and α-hydromuconic acid.

Example 16

Test for production of 3-hydroxyadipic acid and α-hydromuconic acid using mutant *Escherichia* microorganism with increased expression of FDH1, Mdh, and PFL, and with introduced plasmid expressing enzymes that catalyze reactions A, B, D and E

Ec/3HAmdh_fdh_PFL prepared in Example 12 was cultured in the same manner as in Reference Example 12. The concentrations of 3-hydroxyadipic acid and α-hydromuconic acid accumulated in the culture supernatant, and of sugars remaining in the culture medium without being used were measured, and the yields of 3-hydroxyadipic acid and α-hydromuconic acid calculated are as presented in Table 2. It was demonstrated that the mutant *Escherichia* microorganisms with increased expression of FDH1, Mdh, and PFL, and with an introduced plasmid expressing enzymes that catalyze the reactions A, B, D and E showed further increased yields of 3-hydroxyadipic acid and α-hydromuconic acid.

Example 17

Test for production of 3-hydroxyadipic acid and α-hydromuconic acid using mutant *Escherichia* microorganism with increased expression of FDH1, Mdh, and PFL, and with reduced function of pyruvate dehydrogenase complex transcriptional repressor and with introduced plasmid expressing enzymes that catalyze reactions A, B, D and E

EcΔR/3HAmdh_fdh_PFL prepared in Example 13 was cultured in the same manner as in Reference Example 12. The concentration of 3-hydroxyadipic acid and α-hydromuconic acid accumulated in the culture supernatant, and of sugars remaining in the culture medium without being used were measured, and the yields of 3-hydroxyadipic acid and α-hydromuconic acid calculated is as presented in Table 2. It was demonstrated that the mutant *Escherichia* microorganisms with increased expression of FDH1, Mdh, and PFL, and with reduced function of pyruvate dehydrogenase complex transcriptional repressor, and with an introduced plasmid expressing enzymes that catalyze the reactions A, B, D and E showed further increased yields of 3-hydroxyadipic acid and α-hydromuconic acid.

Example 18

Test for production of 3-hydroxyadipic acid and α-hydromuconic acid using mutant *Escherichia* microorganism with increased expression of FDH1 and Mdh, and after introduction of plasmid for expression of Lpd with reduced sensitivity to NADH and enzymes that catalyze reactions A, B, D and E

Ec/3HAmdh_fdh_LPD_E354K prepared in Example 5 was cultured in the same manner as in Reference Example 12. The concentrations of 3-hydroxyadipic acid and α-hydromuconic acid accumulated in the culture supernatant, and of sugars remaining in the culture medium without being used were measured, and the yields of 3-hydroxyadipic acid and α-hydromuconic acid calculated are as presented in Table 2. It was demonstrated that the mutant *Escherichia* microorganisms with increased expression of FDH1 and Mdh, and after introduction of plasmid for expression of Lpd with reduced sensitivity to NADH and enzymes that catalyze reactions A, B, D and E showed further increased yields of 3-hydroxyadipic acid and α-hydromuconic acid.

[Table 2]

| | strain | 3HA yield (%) | IIMA yield (%) |
|---|---|---|---|
| Reference Example 12 | Ec/3HApCDF_pMW | 6.1 | 0.017 |
| Comparative Example 6 | Ec/3HAmdh_pMW | 5.2 | 0.017 |
| Example 6 | Ec/3HAmdh_KpLPD | 7.6 | 0.029 |
| Example 15 | Ec/3HAmdh_fdh_KpLPD | 9.3 | 0.033 |
| Example 7 | Ec/3HAmdh_PFL | 6.3 | 0.020 |
| Example 16 | Ec/3HAmdh_fdh_PFL | 10.8 | 0.034 |
| Example 17 | EcΔR/3HAmdh_fdh_PFL | 18.2 | 0.042 |
| Example 8 | Ec/3HAmdh_LPDE354K | 6.7 | 0.018 |
| Example 18 | Ec/3HAmdh_fdh_LPDE354K | 9.8 | 0.030 |

## Claims

1. A genetically modified microorganism having an ability to produce 3-hydroxyadipic acid and/or α-hydromuconic acid, wherein the reaction to generate malic acid from oxaloacetic acid is enhanced, and the reaction to generate acetyl-CoA from pyruvic acid is enhanced.

2. The genetically modified microorganism of claim 1, wherein the enhancement of the reaction to generate malic acid from oxaloacetic acid is an enhancement of the reaction catalyzed by malate dehydrogenase.

3. The genetically modified microorganism of claim 1 or 2, wherein the enhancement of the reaction to generate acetyl-CoA from pyruvic acid is an enhancement of the reaction catalyzed by pyruvate dehydrogenase complex and/or an enhancement of the reaction catalyzed by pyruvate formate-lyase.

4. The genetically modified microorganism of claim 3, wherein the enhancement of the reaction catalyzed by the pyruvate dehydrogenase complex is an enhancement by increased expression of the pyruvate dehydrogenase complex and/or increased activity of the pyruvate dehydrogenase complex.

5. The genetically modified microorganism of claim 4, wherein the increased expression of the pyruvate dehydrogenase complex is achieved by reducing the function of transcriptional repressor of the pyruvate dehydrogenase complex.

6. The genetically modified microorganism of claim 4, wherein the increased activity of the pyruvate dehydrogenase complex is achieved by reducing the sensitivity of the pyruvate dehydrogenase complex to NADII.

7. The genetically modified microorganism of claim 3, wherein the enhancement of the reaction catalyzed by pyruvate formate-lyase is achieved by enhancement by increased expression of pyruvate formate-lyase.

8. The genetically modified microorganism of any one of claims 1 to 7, wherein the reaction to generate carbon dioxide from formic acid is enhanced.

9. The genetically modified microorganism of claim 8, wherein the enhancement of the reaction to generate carbon dioxide from formic acid is an enhancement of the reaction catalyzed by $NAD^+$-dependent formate dehydrogenase.

10. The genetically modified microorganism of any one of claims 1 to 9, wherein the reaction that reduces 3-oxoadipyl-CoA to generate 3-hydroxyadipyl-CoA is further enhanced.

11. The genetically modified microorganism of any one of claims 1 to 10, wherein the microorganism is one which does not have glucose metabolism via the phosphoketolase pathway.

12. A method of producing 3-hydroxyadipic acid and/or α-hydromuconic acid, comprising the step of culturing the genetically modi fied microorganism of any one of claims 1 to 11.

13. A method of producing adipic acid, comprising the step of producing 3-hydroxyadipic acid and/or α-hydromuconic acid by the process of claim 12, and the step of allowing the 3-hydroxyadipic acid and/or α-hydromuconic acid to react with hydrogen in the presence of a hydrogenation catalyst.

14. A method of producing polyamide, comprising the step of producing adipic acid by the method of claim 13, and the step of polycondensation of the adipic acid and a diamine.

15. The method of producing polyamide of claim 14, wherein the diamine is one containing 1,4-butanediamine, 1,5-pentanediamine, or hexamethylenediamine.

## INTERNATIONAL SEARCH REPORT

International application No.
**PCT/JP2023/004887**

**A. CLASSIFICATION OF SUBJECT MATTER**

***C12P 7/42***(2006.01)i; ***C12N 1/21***(2006.01)i; ***C12N 9/02***(2006.01)i; ***C12N 9/04***(2006.01)i; ***C12N 15/53***(2006.01)i; ***C12N 15/60***(2006.01)i
FI: C12P7/42; C12N15/53; C12N15/60; C12N9/02; C12N9/04 Z; C12N1/21

According to International Patent Classification (IPC) or to both national classification and IPC

**B. FIELDS SEARCHED**

Minimum documentation searched (classification system followed by classification symbols)

C12P7/42; C12N1/21; C12N9/02; C12N9/04; C12N15/53; C12N15/60

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII); CAplus/REGISTRY/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| Y | WO 2020/230719 A1 (TORAY INDUSTRIES, INC) 19 November 2020 (2020-11-19) claims, examples | 1-7, 10-15 |
| Y | WO 2019/107516 A1 (TORAY INDUSTRIES, INC) 06 June 2019 (2019-06-06) claims, examples | 1-7, 10-15 |
| Y | CN 113046283 A (JIANGNAN UNIVERSITY) 29 June 2021 (2021-06-29) claims, examples, fig. 1, 2, 4 | 1-7, 10-15 |
| Y | HAO, T. et al. Engineering the Reducive TCA Pathway to Dynamically Regulate the Biosynthesis of Adipic Acid in Escherichia coli. ACS Synth. Biol. 2021, vol. 10, pp. 632-639 abstract, fig. 1, 4 | 1-7, 10-15 |
| Y | WO 2013/163292 A2 (BIOAMBER INC.) 31 October 2013 (2013-10-31) claims, paragraphs [0091], [0092] | 1-7, 10-15 |
| Y | WO 2014/099725 A1 (GENOMATICA, INC.) 26 June 2014 (2014-06-26) claims, examples | 1-7, 10-15 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **25 April 2023** | **23 May 2023** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (January 2015)

**INTERNATIONAL SEARCH REPORT**

International application No.
**PCT/JP2023/004887**

**C. DOCUMENTS CONSIDERED TO BE RELEVANT**

| Category* | Citation of document, with indication, where appropriate, of the relevant passages | Relevant to claim No. |
|---|---|---|
| Y | JP 2012-525856 A (GENOMATICA, INC.) 25 October 2012 (2012-10-25) claims, examples | 1-7, 10-15 |
| Y | SIRASANI, G. et al. A Biocompatible Alkene Hydrogenation Merges Organic Synthesis with Microbial Metabolism. Angew. Chem. Int. Ed. 2014, vol. 53, pp. 7785-7788 abstract, scheme 1. | 13-15 |
| Y | JP 2009-191156 A (MITSUBISHI CHEMICALS CORP) 27 August 2009 (2009-08-27) claims, examples | 14, 15 |
| Y | CN 112695048 A (ENZYMASTER (NINGBO) BIO-ENGINEERING CO., LTD.) 23 April 2021 (2021-04-23) claims, examples | 14, 15 |
| Y | JP 2008-505652 A (DSM IP ASSETS B.V) 28 February 2008 (2008-02-28) claims, examples | 14, 15 |
| Y | WO 2013/015212 A1 (TORAY INDUSTRIES, INC) 31 January 2013 (2013-01-31) paragraph [0038] | 14, 15 |
| A | WO 2016/044713 A1 (GENOMATICA, INC.) 24 March 2016 (2016-03-24) example VII | 1-15 |
| P, A | WO 2022/102635 A1 (TORAY INDUSTRIES, INC) 19 May 2022 (2022-05-19) entire text | 1-15 |

Form PCT/ISA/210 (second sheet) (January 2015)

**INTERNATIONAL SEARCH REPORT**

International application No.
**PCT/JP2023/004887**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   - a. ☑ forming part of the international application as filed:
     - ☑ in the form of an Annex C/ST.25 text file.
     - ☐ on paper or in the form of an image file.
   - b. ☐ furnished together with the international application under PCT Rule 13*ter*.1(a) for the purposes of international search only in the form of an Annex C/ST.25 text file.
   - c. ☐ furnished subsequent to the international filing date for the purposes of international search only:
     - ☐ in the form of an Annex C/ST.25 text file (Rule 13*ter*.1(a)).
     - ☐ on paper or in the form of an image file (Rule 13*ter*.1(b) and Administrative Instructions, Section 713).
2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.
3. Additional comments:

"In the form of an Annex C/ST.25 text file" above should be understood as "in ST.26 format".

Form PCT/ISA/210 (continuation of first sheet) (January 2015)

**INTERNATIONAL SEARCH REPORT**
**Information on patent family members**

International application No.
**PCT/JP2023/004887**

| Patent document cited in search report | Publication date (day/month/year) | Patent family member(s) | Publication date (day/month/year) |
|---|---|---|---|
| WO 2020/230719 A1 | 19 November 2020 | US 2022/0228178 A1<br>claims, examples<br>EP 3967763 A1<br>CN 113795589 A | |
| WO 2019/107516 A1 | 06 June 2019 | US 2020/0291435 A1<br>claims, examples<br>EP 3719121 A1<br>CN 111386339 A | |
| CN 113046283 A | 29 June 2021 | (Family: none) | |
| WO 2013/163292 A2 | 31 October 2013 | US 2013/0288320 A1<br>claims, paragraphs [0092], [0093] | |
| WO 2014/099725 A1 | 26 June 2014 | US 2015/0329885 A1<br>claims, examples<br>EP 2931874 A1<br>CN 104995293 A | |
| JP 2012-525856 A | 25 October 2012 | US 2010/0317069 A1<br>claims, examples<br>WO 2010/129936 A1<br>EP 2427544 B1<br>KR 10-2018-0135985 A | |
| JP 2009-191156 A | 27 August 2009 | (Family: none) | |
| CN 112695048 A | 23 April 2021 | (Family: none) | |
| JP 2008-505652 A | 28 February 2008 | US 2009/0275093 A1<br>claims, examples<br>WO 2006/005604 A1<br>EP 2236613 A1<br>CN 101006183 A | |
| WO 2013/015212 A1 | 31 January 2013 | US 2014/0171614 A1<br>paragraph [0044]<br>EP 2735615 A1<br>CN 103649322 A | |
| WO 2016/044713 A1 | 24 March 2016 | EP 3741865 A1<br>CN 107208118 A<br>US 2017/0298363 A1<br>EP 3194604 B1 | |
| WO 2022/102635 A1 | 19 May 2022 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2019107516 A
- WO 2020230718 A
- WO 2020230719 A
- US 20170298363 A1
- US 2020291435 A1
- JP 61024555 B
- JP 2000508305 A
- WO 2019208427 A
- WO 2021006257 A


### Non-patent literature cited in the description


- Escherichia microorganisms are those having an ability to produce 3-hydroxyadipic acid and α-hydromuconic acid.. *Biotechnol Lett*, December 2011, vol. 33 (12), 2439-44
- **KIM et al.** *J. Bacteriol.*, 2008, vol. 190, 3851-3858
- *Proc Natl Acad Sci U.S.A.*, 06 June 2000, vol. 97 (12), 6640-6645
- *Biosci Biotechnol Biochem.*, December 2007, vol. 71 (12), 2905-11
- *Journal of Molecular Biology*, 1970, vol. 53, 159
- **NM CALVIN** ; **PC HANAWALT**. *J. Bacteriol*, 1988, vol. 170, 2796-2801
- Polyamide Resin Handbook,'' Nikkan Kogyo Shimbun. January 1998
- **ME KOVACH**. *Gene*, 1995, vol. 166, 175-176